# EUROPEAN PATENT SPECIFICATION

(11) **EP 2 201 000 B1**
(45) Date of publication and mention of the grant of the patent: **31.08.2011**
(21) Application number: 08841505.4
(22) Date of filing: 24.10.2008
(51) Int. Cl.: C07D 401/04, C07D 401/14, C07D 409/04, C07D 409/14, A01N 43/50, A61P 35/00, A61K 31/4439

(54) **NOVEL IMIDAZOLE DERIVATIVES**
NEUE IMIDAZOLDERIVATE
NOUVEAUX DÉRIVÉS D'IMIDAZOLE

(30) Priority: 26.10.2007 EP 07020982; 17.12.2007 EP 07024449
(43) Date of publication of application: 30.06.2010
(73) Proprietor: Syngenta Participations AG, 4058 Basel (CH)
(72) Inventor: DUMEUNIER, Raphael, CH-4332 Stein (CH); LAMBERTH, Clemens, CH-4332 Stein (CH); TRAH, Stephan, CH-4332 Stein (CH); WENDEBORN, Sebastian, CH-4332 Stein (CH)
(74) Representative: Herrmann, Jörg
(86) International application number: PCT/EP2008/009043
(87) International publication number: WO 2009/053102

(56) References cited:
- WO-A-2004/052280
- US-A1- 2002 022 728

## Description

The present invention relates to novel imidazole derivatives as active ingredients which have microbiocidal activity, in particular fungicidal activity. The invention also relates to preparation of these active ingredients, to novel heterocyclic derivatives used as intermediates in the preparation of these active ingredients, to preparation of these novel intermediates, to agrochemical compositions which comprise at least one of the novel active ingredients, to preparation of these compositions and to use of the active ingredients or compositions in agriculture or horticulture for controlling or preventing infestation of plants, harvested food crops, seeds or non-living materials by phytopathogenic microorganisms, preferably fungi.

US2002022728 relates to nitrophenyl-sulphonyl-imidazoles for controlling vegetable and animal pests.WO2004052280 relates to compounds that inhibit angiogenesis and are useful in the treatment of angiogenic dependent diseases like cancer.

In addition, the present invention also relates to the use of these novel imidazole derivatives as plant growth regulators (PGRs).

Furthermore, the present invention also relates to compositions comprising novel imidazole derivatives, that improve plants, a process which is commonly and hereinafter referred to as "plant health".

The present invention further relates to fungicidal compositions comprising at least one of these compounds as active component.

These objects are achieved by the following compound of formula I: 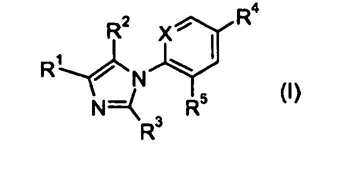 wherein
R¹ is halogen, C₁-C₄alkyl or C₁-C₄haloalkyl;
R² is an optionally substituted aryl or heteroaryl;
R³ is halogen;
R⁴ is hydrogen, halogen, C₁-C₄alkyl, C₁-C₄haloalkyl, C₁-C₄alkoxy, C₁-C₄haloalkoxy or cyano;
R⁵ is halogen;
X is N or C(R); and
R is hydrogen, halogen, C₁-C₄alkyl, C₁-C₄haloalkyl, C₁-C₄alkoxy, C₁-C₄haloalkoxy or cyano; or an agrochemically usable salt form thereof;
provided that
when X is C(R), R² cannot be an optionally substituted aryl.

In the above definition aryl includes aromatic hydrocarbon rings like phenyl, naphthyl, anthracenyl, phenanthrenyl and biphenyl, with phenyl being preferred.

Heteroaryl stands for aromatic ring systems comprising mono-, bi- or tricyclic systems wherein at least one oxygen, nitrogen or sulfur atom is present as a ring member. Examples are furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, benzoxazolyl, quinolyl, isoquinolyl, phthalazinyl, quinoxalinyl, quinazolinyl, cinnolinyl and naphthyridinyl.

The above or below mentioned fused ring, carbocyclic ring, heterocyclic ring, aryl group and heteroaryl group may be optionally substituted. This means that they may carry one or more identical or different substituents. Normally not more than three substituents are present at the same time. Examples of substituents are: halogen, alkyl, haloalkyl, cycloalkyl, cycloalkylalkyl, alkenyl, haloalkenyl, cycloalkenyl, alkynyl, haloalkynyl, alkyloxy, haloalkyloxy, cycloalkoxy, alkenyloxy, haloalkenyloxy, alkynyloxy, haloalkenyloxy, alkylthio, haloalkylthio, cycloalkylthio, alkenylthio, alkynylthio, alkylcarbonyl, haloalkylcarbonyl, cycloalkylcarbonyl, alkenylcarbonyl, alkynylcarbonyl, alkoxyalkyl, cyano, nitro, hydroxy, mercapto, amino, alkylamino, dialkylamino. Typical examples for optionally substituted aryl include 2-fluorophenyl, 3-fluorophenyl, 4-fluorophenyl, 2-chlorophenyl, 3-chlorophenyl, 4-chlorophenyl, 3-bromophenyl, 4-bromophenyl, m-tolyl, p-tolyl, 3-trifluoromethylphenyl, 4-trifluoromethylphenyl, 3-methoxyphenyl, 4-methoxyphenyl, 3-trifluoromethoxyphenyl, 4-trifluoromethoxyphenyl, 3-cyanophenyl, 4-cyanophenyl, 2,4-difluorophenyl, 2,5-difluorophenyl, 2,6-difluorophenyl, 3,4-difluorophenyl, 2,4-dichlorophenyl, 2,5-dichlorophenyl, 2,6-dichlorophenyl, 3,4-dichlorophenyl, 3,4-dimethylphenyl, 3,4-dimethoxyphenyl, 2-chloro-4-fluorophenyl, 2-chloro-5-fluorophenyl, 2-chloro-6-fluorophenyl, 3-chloro-4-fluorophenyl, 3-chloro-6-fluorophenyl, 3-chloro-4-methylphenyl, 3-chloro-4-methoxyphenyl, 4-chloro-2-fluorophenyl, 4-chloro-3-fluorophenyl, 4-chloro-3-methylphenyl, 4-chloro-3-methoxyphenyl, 3-fluoro-4-methoxyphenyl, 3-fluoro-4-methylphenyl, 4-fluoro-3-methoxyphenyl, 4-fluoro-3-methylphenyl, 3-methoxy-4-methylphenyl, 4-methoxy-3-methylphenyl, 2,6-diftuoro-4-methylphenyl, 2,6-difluoro-4-trifluoromethylphenyl, 2,6-difluoro-4-methoxyphenyl, 2,6-difluoro-4-trifluoromethoxyphenyl, 2,6-difluoro-4-cyanophenyl, 2,4,6-trifluorophenyl, 2,5,6-trifluorophenyl. Typical examples for optionally substituted heteroaryl include 6-chloropyridin-2-yl, 6-fluoropyridin-2-yl, 6-methoxypyridin-2-yl, 6-methylpyridin-2-yl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, 3,5-dichloropyridin-2-yl, 3,5-difluoropyridin-2-yl, 3-chloro-5-fluoropyridin-2-yl, 3-chloro-5-methylpyridin-2-yl, 3-chloro-5-trifluoromethylpyridin-2-yl, 3-chloro-5-methoxypyridin-2-yl, 3-chloro-5-trifluoromethoxypyridin-2-yl, 3-chloro-5-cyanopyridin-2-yl, 5-chloro-3-fluoropyridin-2-yl, 3-fluoro-5-methylpyridin-2-yl, 3-fluoro-5-trifluoromethylpyridin-2-yl, 3-fluoro-5-methoxypyridin-2-yl, 3-fluoro-5-trifluoromethoxypyridin-2-yl, 3-fluoro-5-cyanopyridin-2-yl, 5-chlorothiophen-2-yl, 5-bromothiophen-2-yl, 5-methoxythiophen-2-yl, 4-methoxyquinolin-2-yl, 4-methylquinolin-2-yl.

In the above definition halogen is fluorine, chlorine, bromine or iodine.

The alkyl, alkenyl or alkynyl radicals may be straight-chained or branched.

Alkyl on its own or as part of another substituent is, depending upon the number of carbon atoms mentioned, for example, methyl, ethyl, propyl, butyl, pentyl, hexyl and the isomers thereof, for example, isopropyl, isobutyl, sec-butyl, tert-butyl, isopentyl or tert-pentyl.

A haloalkyl group may contain one or more identical or different halogen atoms and, for example, may stand for CH₂Cl, CHCl₂, CCl₃, CH₂F, CHF₂, CF₃, CF₃CH₂, CH₃CF₂, CF₃CF₂ or CCl₃CCl₂.

Cycloalkyl on its own or as part of another substituent is, depending upon the number of carbon atoms mentioned, for example, cyclopropyl, cyclobutyl, cyclopentyl or cyclohexyl.

Alkenyl on its own or as part of another substituent is, depending upon the number of carbon atoms mentioned, for example, ethenyl, allyl, 1-propenyl, buten-2-yl, buten-3-yl, penten-1-yl, penten-3-yl, hexen-1-yl or 4-methyl-3-pentenyl.

Alkynyl on its own or as part of another substituent is, depending upon the number of carbon atoms mentioned, for example, ethynyl, propyn-1-yl, propyn-2-yl, butyn-1-yl, butyn-2-yl, 1-methyl-2-butynyl, hexyn-1-yl or 1-ethyl-2-butynyl.

The presence of one or more possible asymmetric carbon atoms in a compound of formula I means that the compounds may occur in optically isomeric, that means enantiomeric or diastereomeric forms. As a result of the presence of a possible aliphatic C=C double bond, geometric isomerism, that means cis-trans or (E)-(Z) isomerism may also occur. Also atropisomers may occur as a result of restricted rotation about a single bond. Formula I is intended to include all those possible isomeric forms and mixtures thereof. The present invention intends to include all those possible isomeric forms and mixtures thereof for a compound of formula I.

In each case, the compounds of formula I according to the invention are in free form or in an agronomically usable salt form.

In a first embodiment, compounds of formula I according to the invention have R¹ which is halogen, C₁-C₃alkyl or C₁-C₃haloalkyl.

In a second embodiment, compounds of formula I according to the invention have R² which is an optionally substituted phenyl, naphthyl, pyridyl, thienyl or quinolyl.

In a third embodiment, compounds of formula I according to the invention have R³ which is fluoro, chloro, bromo or iodo.

In a fourth embodiment, compounds of formula I according to the invention have R⁴ which is hydrogen, halogen, C₁-C₃alkyl, C₁-C₃haloalkyl, C₁-C₃alkoxy, C₁-C₃haloalkoxy or cyano.

In a fifth embodiment, compounds of formula I according to the invention have R⁵ which is fluoro, chloro, bromo or iodo.

In a sixth embodiment, compounds of formula I according to the invention have X which is N, C(H), C(halogen), C(C₁-C₄alkyl), C(C₁-C₄haloalkyl), C(C₁-C₄alkoxy) or C(C₁-C₄haloalkoxy).

Preferred subgroups of compounds of formula I according to the invention are those wherein R¹ is fluoro, chloro, bromo, iodo, C₁-G₂alkyl or C₁-C₂haloalkyl;
R² is an optionally substituted phenyl, pyridyl, thienyl or quinolyl;
R³ is fluoro, chloro or bromo;
R⁴ is hydrogen, fluoro, chloro, bromo, C₁-C₃alkyl, C₁-C₃haloalkyl, C₁-C₃alkoxy, C₁-C₃haloalkoxy or cyano;
R⁵ is fluoro, chloro or bromo; and
X is N, C(H), C(Cl), C(F), C(Br), C(I), C(C₁-C₃alkyl), C(C₁-C₃haloalkyl), C(C₁-C₃alkoxy) or C(C₁-C₃haloalkoxy).

More preferred subgroups of compounds of formula I according to the invention are those wherein
R¹ is fluoro, chloro, bromo, methyl or ethyl;
R² is phenyl, 3-fluorophenyl, 4-fluorophenyl, 3-chlorophenyl, 4-chlorophenyl, 3-bromophenyl, 4-bromophenyl, m-tolyl, p-tolyl, 3-trifluoromethylphenyl, 4-trifluoromethylphenyl, 3-methoxyphenyl, 4-methoxyphenyl, 3-trifluoromethoxyphenyl, 4-trifluoromethoxyphenyl, 3-cyanophenyl, 4-cyanophenyl, 3,4-difluorophenyl, 3,4-dichlorophenyl, 3,4-dimethylphenyl, 3,4-dimethoxyphenyl, 3-chloro-4-fluorophenyl, 3-chloro-4-methylphenyl, 3-chloro-4-methoxyphenyl, 4-chloro-3-fluorophenyl, 4-chloro-3-methylphenyl, 4-chloro-3-methoxyphenyl, 3-fluoro-4-methoxyphenyl, 3-fluoro-4-methylphenyl, 4-fluoro-3-methoxyphenyl, 4-fluoro-3-methylphenyl, 3-methoxy-4-methylphenyl, 4-methoxy-3-methylphenyl, pyridin-2-yl, pyridin-3-yl, pyridin-4-yl, 6-chloropyridin-2-yl, 6-fluoropyridin-2-yl, 6-methoxypyridin-2-yl, 6-methylpyridin-2-yl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, 5-chlorothiophen-2-yl, 5-bromothiophen-2-yl, 5-methoxythiophen-2-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl, C₁-C₂alkoxy, C₁-C₂haloalkoxy or cyano;
R⁵ is fluoro or chloro; and
X is N, C(H), C(Cl), C(F), C(Br), C(C₁-C₂alkyl), C(C₁-C₂haloalkyl), C(C₁-C₂alkoxy) or C(C₁-C₂haloalkoxy).

Most preferred subgroups of compounds of formula I according to the invention are those wherein
R¹ is fluoro, chloro, methyl or ethyl;
R² is 4-fluorophenyl, 4-chlorophenyl, 4-bromophenyl, 6-chloropyridin-3-yl, 6-fluoropyridin-3-yl, 6-methoxypyridin-3-yl, 6-methylpyridin-3-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl or C₁-C₂alkoxy;
R⁵ is fluoro or chloro; and
X is N, C(H), C(Cl) or C(F).

Especially preferred subgroups of compounds of formula I according to the invention are those wherein
R¹ is chloro or methyl;
R² is 4-bromophenyl, 6-chloropyridin-3-yl or quinolin-3-yl;
R³ is chloro;
R⁴ is chloro;
R⁵ is chloro; and
X is N.

Preferred individual compounds are:
2-Chloro-5-[2,5-dichloro-3-(2,4,6-trifluoro-phenyl)-3H-imidazol-4-yl]-pyridine;
2-Chloro-5-[2-chloro-5-methyl-3-(2,4,6-trifluoro-phenyl)-3H-imidazol-4-yl]-pyridine;
5-[2-Chloro-5-methyl-3-(2,4,6-trifluoro-phenyl)-3H-imidazol-4-yl]-2-methoxy-pyridine;
3-[2-Chloro-5-methyl-3-(2,4,6-trifluoro-phenyl)-3H-imidazol-4-yl]-quinoline;
3-[2,5-Dichloro-3-(2,4,6-trifluoro-phenyl)-3H-imidazol-4-yl]-quinoline;
3-[2-Chloro-3-(2,6-difluoro-4-methoxy-phenyl)-5-methyl-3H-imidazol-4-yl]-quinoline;
3-[2,5-Dichloro-3-(2,6-difluoro-4-methoxy-phenyl)-3H-imidazol-4-yl]-quinoline;
2-[2-Chloro-5-methyl-3-(2,4,6-trifluoro-phenyl)-3H-imidazol-4-yl]-quinoline;
2-[2-Chloro-5-methyl-3-(2,4,6-trifluoro-phenyl)-3H-imidazol-4-yl]-4-methoxy-quinoline;
2-[2-Chloro-3-(2,6-difluoro-4-methoxy-phenyl)-5-methyl-3H-imidazol-4-yl]-4-methoxy-quinoline;
3,5-Dichloro-2-[2,4-dichloro-5-(6-chloro-pyridin-3-yl)-imidazol-1-yl]-pyridine;
3,5-Dichloro-2-[2-chloro-5-(6-chloro-pyridin-3-yl)-4-methyl-imidazol-1-yl]-pyridine;
5-[2-Chloro-3-(2,6-difluoro-4-methoxy-phenyl)-5-methyl-3H-imidazol-4-yl]-2-methyl-pyridine; and
3,5-Dichloro-2-[2-chloro-5-(4-methoxy-phenyl)-4-methyl-imidazol-1-yl]-pyridine.

Compounds of formula (I.1), 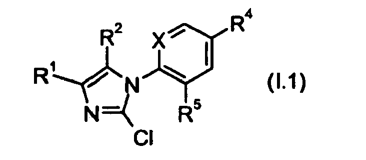 in which R¹, R², R⁴, R⁵ and X have the meanings given above, provided that when X is C(R), R² cannot be an optionally substituted aryl, are examples of compounds of general formula (I) and can be made as shown in the following schemes.

The compounds of formula 1.1, wherein R², R⁴, R⁵ and X are as defined for compound of formula I, provided that when X is C(R), R² cannot be an optionally substituted aryl, and R¹ is C₁-C₄alkyl or C₁-C₄haloalkyl, can be obtained by reaction of a compound of formula II, wherein R², R⁴, R⁵ and X are as defined for compound of formula I, provided that when X is C(R), R² cannot be an optionally substituted aryl, and R¹ is C₁-C₄alkyl or C₁-C₄haloalkyl, with N-chlorosuccinimide (NCS) or molecular chlorine.

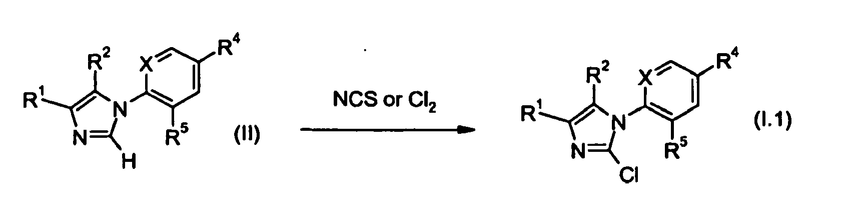

The compounds of formula I, wherein R², R³, R⁴, R⁵ and X are as defined for compound of formula I, provided that when X is C(R), R² cannot be an optionally substituted aryl, and R¹ is Halogen, preferably chlorine or bromine, can be obtained by reaction of a compound of formula III, wherein R², R⁴, R⁵ and X are as defined for compound of formula I, provided that when X is C(R), R² cannot be an optionally substituted aryl, with at least 2 equivalents of N-chlorosuccinimide (NCS), N-bromosuccinimide (NBS) or N-iodosuccinimide (NIS).

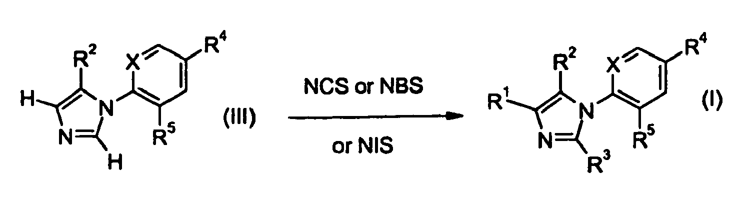

The compounds of formula 1.1, wherein R², R⁴, R⁵ and X are as defined for compound of formula I, provided that when X is C(R), R² cannot be an optionally substituted aryl, and R¹ is C₁-C₄haloalkyl, can be obtained by reaction of a compound of formula II, wherein R², R⁴, R⁵ and X are as defined for compound of formula I, provided that when X is C(R), R² cannot be an optionally substituted aryl, and R¹ is C₁-C₄alkyl, with at least 2 equivalents of N-chlorosuccinimide (NCS) or molecular chlorine.

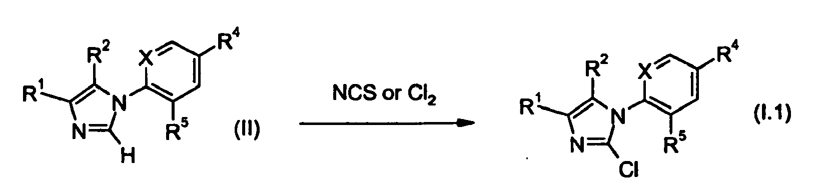

The compounds of formula II, wherein R², R⁴, R⁵ and X are as defined for compound of formula I, provided that when X is C(R), R² cannot be an optionally substituted aryl, and R¹ is C₁-C₄alkyl, can be obtained by reaction of a compound of formula IV, wherein R², R⁴, R⁵ and X are as defined for compound of formula I, provided that when X is C(R), R² cannot be an optionally substituted aryl, with a compound of formula V, wherein R¹ is C₁-C₄alkyl, in the presence of a base, e.g. anhydrous potassium carbonate, as already described in Journal of Medicinal Chemistry 2003, 46, 3463.

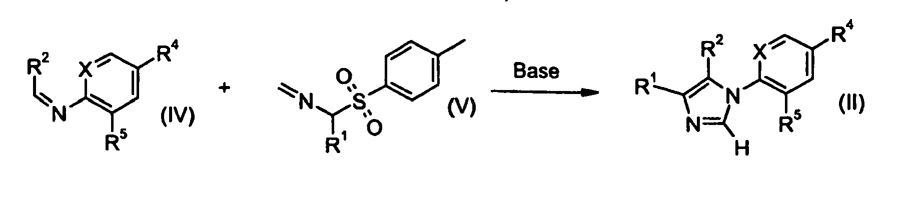

The compounds of formula III, wherein R², R⁴, R⁵ and X are as defined for compound of formula I, provided that when X is C(R), R² cannot be an optionally substituted aryl, can be obtained by reaction of a compound of formula IV, wherein R², R⁴, R⁵ and X are as defined for compound of formula I, provided that when X is C(R), R² cannot be an optionally substituted aryl, with toluenesulfonylmethyl isocyanide, in the presence of a base, e.g. anhydrous potassium carbonate, as already described in Journal of Medicinal Chemistry 2003, 46, 3463.

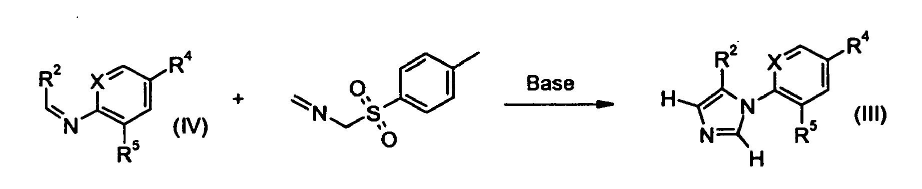

The compounds of formula IV, wherein R², R⁴, R⁵ and X are as defined for compound of formula I, provided that when X is C(R), R² cannot be an optionally substituted aryl, can be obtained by reaction of an aldehyde of formula VI, wherein R² is as defined for compound of formula I, with an amine of formula VII, wherein R⁴, R⁵ and X are as defined for compound of formula I, provided that when X is C(R) in compound of formula VII, R² cannot be an optionally substituted aryl in compound of formula VI, as already described in Journal of Medicinal Chemistry 2003, 46, 3463.

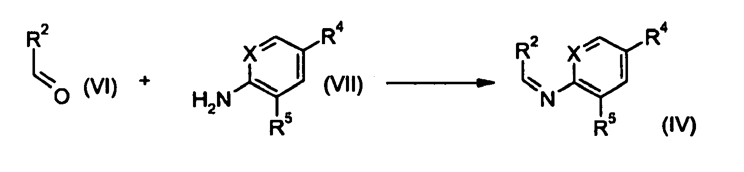

The compounds of formula V, wherein R¹ is C₁-C₄alkyl, can be obtained by reaction of toluenesulfonylmethyl isocyanide, with a compound of formula VIII, wherein R¹ is C₁-C₄alkyl, in the presence of a base, as already described in Journal of Medicinal Chemistry 2003, 46, 3463.

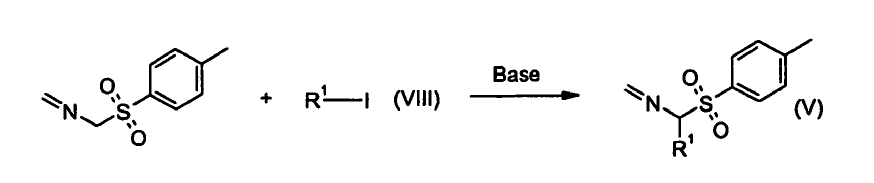

Surprisingly, it has now been found that the novel compounds of formula I have, for practical purposes, a very advantageous level of biological activity for protecting plants against diseases that are caused by fungi as well as by bacteria and viruses.

The compounds of formula I can be used in the agricultural sector and related fields of use as active ingredients for controlling plant pests or on non-living materials for control of spoilage microorganisms or organisms potentially harmfull to man. The novel compounds are distinguished by excellent activity at low rates of application, by being well tolerated by plants and by being environmentally safe. They have very useful curative, preventive and systemic properties and are used for protecting numerous cultivated plants. The compounds of formula I can be used to inhibit or destroy the pests that occur on plants or parts of plants (fruit, blossoms, leaves, stems, tubers, roots) of different crops of useful plants, while at the same time protecting also those parts of the plants that grow later e.g. from phytopathogenic microorganisms.

It is also possible to use compounds of formula I as dressing agents for the treatment of plant propagation material, *e.g*., seed, such as fruits, tubers or grains, or plant cuttings (for example rice), for the protection against fungal infections as well as against phytopathogenic fungi occurring in the soil. The propagation material can be treated with a composition comprising a compound of formula I before planting: seed, for example, can be dressed before being sown. The active ingredients according to the invention can also be applied to grains (coating), either by impregnating the seeds in a liquid formulation or by coating them with a solid formulation. The composition can also be applied to the planting site when the propagation material is being planted, for example, to the seed furrow during sowing. The invention relates also to such methods of treating plant propagation material and to the plant propagation material so treated.

Furthermore the compounds according to present invention can be used for controlling fungi in related areas, for example in the protection of technical materials, including wood and wood related technical products, in food storage, in hygiene management.

In addition, the invention could be used to protect non-living materials from fungal attack, e.g. lumber, wall boards and paint.

The compounds of formula I are, for example, effective against the phytopathogenic fungi of the following classes: Fungi imperfecti (e.g. *Alternaria* spp.), Basidiomycetes (e.g. *Corticium* spp., *Ceratobasidium* spp., *Waitea* spp., *Thanatephorus* spp., *Rhizoctonia* spp., *Hemileia* spp., *Puccinia* spp., *Phakopsora* spp., *Ustilago* spp., *Tilletia* spp.), Ascomycetes (e.g. *Venturia* spp., *Blumeria* spp., *Erysiphe* spp., *Podosphaera* spp., *Uncinula* spp., *Monilinia* spp., *Sclerotinia* spp., *Colletotrichum* spp., *Glomerella* spp., *Fusarium* spp., *Gibberella* spp., *Monographella* spp., *Phaeosphaeria* spp., *Mycosphaerella* spp., *Cercospora* spp., *Pyrenophora* spp., *Rhynchosporium* spp., *Magnaporthe* spp., *Gaeumannomyces* spp., *Oculimacula* spp., *Ramularia* spp., *Botryotinia* spp.) and Oomycetes (e.g. *Phytophthora* spp., *Pythium* spp., *Plasmopara* spp., *Peronospora* spp., *Pseudoperonospora* spp. *Bremia* spp). Outstanding activity has been observed against powdery mildews (e.g. *Uncinula necator*), rusts (e.g. *Puccinia* spp.) and leaf spots (e.g. *Mycosphaerella* spp.). Furthermore, the novel compounds of formula I are effective against phytopathogenic gram negative and gram positive bacteria (e.g. *Xanthomonas* spp, *Pseudomonas* spp, *Erwinia amylovora, Ralstonia* spp.) and viruses (e.g. tobacco mosaic virus).

Within the scope of present invention, useful plants and / or target crops to be protected typically comprise the following species of plants: cereal (wheat, barley, rye, oat, rice, maize, sorghum and related species); beet (sugar beet and fodder beet); pomes, drupes and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamomum, camphor) or plants such as tobacco, nuts, coffee, eggplants, sugar cane, tea, pepper, vines, hops, bananas and natural rubber plants, as well as turf and ornamentals.

The useful plants and / or target crops in accordance with the invention include conventional as well as genetically enhanced or engineered varieties such as, for example, insect resistant (e.g. Bt. and VIP varieties) as well as disease resistant, herbicide tolerant (e.g. glyphosate- and glufosinate-resistant maize varieties commercially available under the trade names RoundupReady® and LibertyLink®) and nematode tolerant varieties. By way of example, suitable genetically enhanced or engineered crop varieties include the Stoneville 5599BR cotton and Stoneville 4892BR cotton varieties.

The terms "useful plants" and / or "target crops" are to be understood as including also useful plants that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors or PPO (protoporphyrinogen-oxidase) inhibitors) as a result of conventional methods of breeding or genetic engineering. An example of a crop that has been rendered tolerant to imidazolinones, e.g. imazamox, by conventional methods of breeding (mutagenesis) is Clearfield® summer rape (Canola). Examples of crops that have been rendered tolerant to herbicides or classes of herbicides by genetic engineering methods include glyphosate- and glufosinate-resistant maize varieties commercially available under the trade names RoundupReady®, Herculex I® and LibertyLink®.

The terms "useful plants" and / or "target crops" are to be understood as including also useful plants which have been so transformed by the use of recombinant DNA techniques that they are capable of synthesising one or more selectively acting toxins, such as are known, for example, from toxin-producing bacteria, especially those of the genus Bacillus.

The terms "useful plants" and / or "target crops" are to be understood as including also useful plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818, and EP-A-0 353 191. The methods of producing such transgenic plants are generally known to the person skilled in the art and are described, for example, in the publications mentioned above.

The term "locus" of a useful plant as used herein is intended to embrace the place on which the useful plants are growing, where the plant propagation materials of the useful plants are sown or where the plant propagation materials of the useful plants will be placed into the soil. An example for such a locus is a field, on which crop plants are growing.

The term "plant propagation material" is understood to denote generative parts of the plant, such as seeds, which can be used for the multiplication of the latter, and vegetative material, such as cuttings or tubers, for example potatoes. There may be mentioned for example seeds (in the strict sense), roots, fruits, tubers, bulbs, rhizomes and parts of plants. Germinated plants and young plants which are to be transplanted after germination or after emergence from the soil, may also be mentioned. These young plants may be protected before transplantation by a total or partial treatment by immersion. Preferably "plant propagation material" is understood to denote seeds.

The compounds of formula I are used in unmodified form or, preferably, together with the adjuvants conventionally employed in the art of formulation. To this end they are conveniently formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of formula I are normally used in the form of compositions and can be applied to the crop area or plant to be treated, simultaneously or in succession with further compounds. These further compounds can be e.g. fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of formula I are normally used in the form of fungicidal compositions for controlling or protecting against phytopathogenic microorganisms, comprising as active ingredient at least one compound of formula I or of at least one preferred individual compound as above-defined, in free form or in agrochemically usable salt form, and at least one of the above-mentioned adjuvants.

Said fungicidal compositions for controlling or protecting against phytopathogenic microorganisms, comprising as active ingredient at least one compound of formula I or of at least one preferred individual compound as above-defined, in free form or in agrochemically usable salt form, and at least one of the above-mentioned adjuvants can be mixed with other fungicides, resulting in some cases in unexpected synergistic activities. Mixing components which are particularly preferred are:
Azoles, such as azaconazole, BAY 14120, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, prothioconazole, pyrifenox, prochloraz, propiconazole, simeconazole, tebuconazole, tetraconazole, triadimefon, triadimenol, triflumizole, triticonazole;
Pyrimidinyl carbinoles, such as ancymidol, fenarimol, nuarimol;
2-amino-pyrimidines, such as bupirimate, dimethirimol, ethirimol;
Morpholines, such as dodemorph, fenpropidine, fenpropimorph, spiroxamine, tridemorph;
Anilinopyrimidines, such as cyprodinil, mepanipyrim, pyrimethanil;
Pyrroles, such as fenpiclonil, fludioxonil;
Phenylamides, such as benalaxyl, furalaxyl, metalaxyl, R-metalaxyl, ofurace, oxadixyl;
Benzimidazoles, such as benomyl, carbendazim, debacarb, fuberidazole, thiabendazole;
Dicarboximides, such as chlozolinate, dichlozoline, iprodione, myclozoline, procymidone, vinclozoline;
Carboxamides, such as boscalid, carboxin, fenfuram, flutolanil, mepronil, oxycarboxin, penthiopyrad, thifluzamide; guanidines, such as guazatine, dodine, iminoctadine;
Strobilurines, such as azoxystrobin, dimoxystrobin, enestroburin, fluoxastrobin, kresoxim-methyl, metominostrobin, trifloxystrobin, orysastrobin, picoxystrobin, pyraclostrobin;
Dithiocarbamates, such as ferbam, mancozeb, maneb, metiram, propineb, thiram, zineb, ziram;
N-halomethylthiotetrahydrophthalimides, such as captafol, captan, dichlofluanid, fluoromides, folpet, tolyfluanid;
Copper-compounds, such as Bordeaux mixture, copper hydroxide, copper oxychloride, copper sulfate, cuprous oxide, mancopper, oxine-copper;
Nitrophenol-derivatives, such as dinocap, nitrothal-isopropyl;
Organo-phosphorus-derivatives, such as edifenphos, iprobenphos, isoprothiolane, phosdiphen, pyrazophos, tolclofos-methyl;
Pyridazine-derivatives which are known and may be prepared by methods as described in WO 05/121104, WO 06/001175 and WO 07/066601, such as 3-chloro-5-(4-chloro-phenyl)-6-methyl-4-(2,4,6-trifluoro-phenyl)-pyridazine (formula P.1), 3-chloro-6-methyl-5-p-tolyl-4-(2,4,6-trifluoro-phenyl)-pyridazine (formula P.2) and 3-chloro-4-(3-chloro-5-methoxy-pyridin-2-yl)-5-(4-chloro-phenyl)-6-methyl-pyridazine (formula P.3); 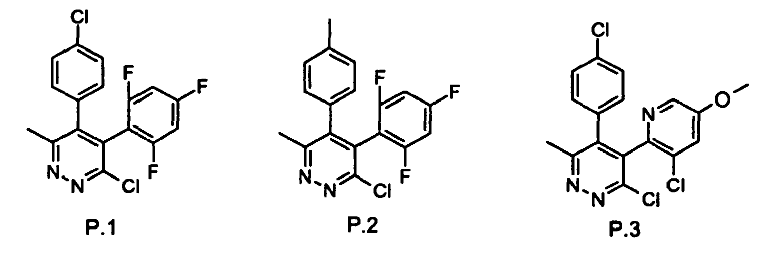
Triazolopyrimidine derivatives which are known and may be prepared by methods as described in WO98/46607, such as 5-chloro-7-(4-methyl-piperidin-1-yl)-6-(2,4,6-trifluorophenyl)- [1,2,4]triazolo[1,5-a]pyrimidine (formula T.1); 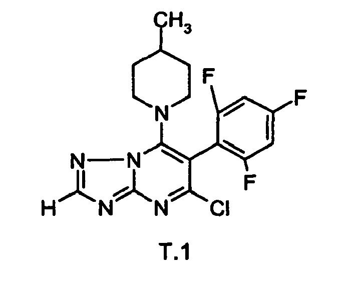
Carboxamide derivatives which are known and may be prepared by methods as described in WO04/035589, WO06/37632, WO03/074491 or WO03070705, such as 3-difluoromethyl-1-methyl-1H-pyrazole-4-carboxylic acid (9-isopropyp-1,2,3,4-tetrahaydro-1,4-methano-naphthalen-5-yl)-amide (formula U.1), 3-difluoromethyl-1-methyl-1H-pyrazole-4-carboxylic acid (2-bicyclopropyl-2-yl-phenyl)-amide (formula U.2) or N-(3',4'-dichloro-5-fluoro-1,1'-biphenyl-2-yl)-3-(difluoromethyl)-1-methyl-1H-pyrazole-4-carboxamide; 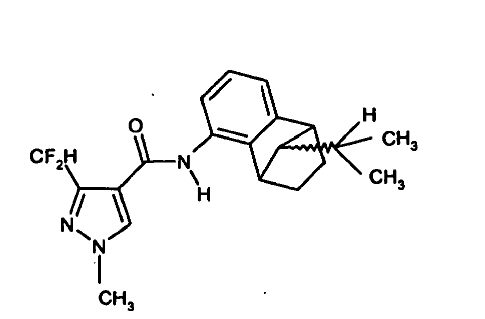 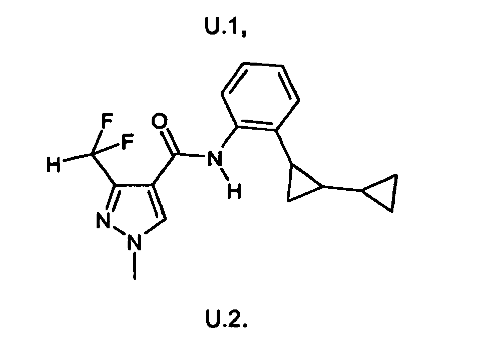
Benzamide derivatives which are known and may be prepared by methods as described in WO 2004/016088, such as N-{-2-[3-chloro-5-(trifluoromethyl)-2-pyridinyl]ethyl}-2-trifluoromethylbenzamide, which is also known under the name fluopyram (formula V.1); 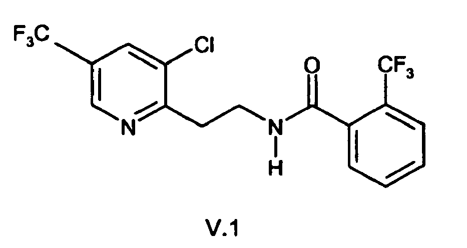 and
Various others, such as acibenzolar-S-methyl, anilazine, benthiavalicarb, blasticidin-S, chinomethionate, chloroneb, chlorothalonil, cyflufenamid, cymoxanil, dichlone, diclocymet, diclomezine, dicloran, diethofencarb, dimethomorph, flumorph, dithianon, ethaboxam, etridiazole, famoxadone, fenamidone, fenoxanil, fentin, ferimzone, fluazinam, fluopicolide, flusulfamide, fenhexamid, fosetyl-aluminium, hymexazol, iprovalicarb, cyazofamid, kasugamycin, mandipropamid, methasulfocarb, metrafenone, nicobifen, pencycuron, phthalide, polyoxins, probenazole, propamocarb, proquinazid, pyroquilon, quinoxyfen, quintozene, sulfur, tiadinil, triazoxide, tricyclazole, triforine, validamycin, zoxamide and glyphosate.

Another aspect of invention is related to the use of a compound of formula I or of a preferred individual compound as above-defined, of a composition comprising at least one compound of formula I or at least one preferred individual compound as above-defined, or of a fungicidal mixture comprising at least one compound of formula I or at least one preferred individual compound as above-defined in admixture with other fungicides, as described above, for controlling or preventing infestation of plants, harvested food crops, seeds or non-living materials by phytopathogenic microorganisms, preferably fungal organisms.

A further aspect of invention is related to a method of controlling or preventing an infestation of crop plants, harvested food crops or of non-living materials by phytopathogenic or spoilage microorganisms or organisms potentially harmful to man, especially fungal organisms, which comprises the application of a compound of formula I or of a preferred individual compound as above-defined as active ingredient to the plants, to parts of the plants or to the locus thereof, to seeds or to any part of the non-living materials.

Controlling or preventing means reducing the infestation of crop plants or of non-living materials by phytopathogenic or spoilage microorganisms or organisms potentially harmful to man, especially fungal organisms, to such a level that an improvement is demonstrated.

A preferred method of controlling or preventing an infestation of crop plants by phytopathogenic microorganisms, especially fungal organisms, which comprises the application of a compound of formula I, or an agrochemical composition which contains at least one of said compounds, is foliar application. The frequency of application and the rate of application will depend on the risk of infestation by the corresponding pathogen. However, the compounds of formula I can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula I may also be applied to seeds (coating) by impregnating the seeds or tubers either with a liquid formulation of the fungicide or coating them with a solid formulation.

A formulation [that is, a composition containing the compound of formula I] and, if desired, a solid or liquid adjuvant or monomers for encapsulating the compound of formula I, is prepared in a known manner, typically by intimately mixing and/or grinding the compound with extenders, for example solvents, solid carriers and, optionally, surface active compounds (surfactants).

The agrochemical formulations will usually contain from 0.1 to 99% by weight, preferably from 0.1 to 95% by weight, of the compound of formula 1, 99.9 to 1% by weight, preferably 99.8 to 5% by weight, of a solid or liquid adjuvant, and from 0 to 25% by weight, preferably from 0.1 to 25% by weight, of a surfactant.

Advantageous rates of application are normally from 5g to 2kg of active ingredient (a.i.) per hectare (ha), preferably from 10g to 1 kg a.i./ha, most preferably from 20g to 600g a.i./ha. When used as seed drenching agent, convenient dosages are from 10mg to 1g of active substance per kg of seeds.

Whereas it is preferred to formulate commercial products as concentrates, the end user will normally use dilute formulations.

Surprisingly, the imidazole compounds of formula I according to the invention, in particular the individual imidazole compounds described in the above description as being preferred, also present a plant growth regulator (PGR) activity. Therefore, the present invention also relates to the use of these novel imidazole derivatives as plant growth regulators (PGRs).

Plant growth regulators (PGRs) are generally any substances or mixtures of substances intended to accelerate or retard the rate of growth or maturation, or otherwise alter the development of plants or their produce.

Plant growth regulators (PGRs) affect growth and differentiation of plants.

More specifically, various plant growth regulators (PGRs) can, for example, reduce plant height, stimulate seed germination, induce flowering, darken leaf coloring, change the rate of plant growth and modify the timing and efficiency of fruiting.

Furthermore, the present invention also relates to compositions comprising the novel imidazole derivatives of the present invention that improve plants, a process which is commonly and hereinafter referred to as "plant health".

For example, advantageous properties that may be mentioned are improved crop characteristics including: emergence, crop yields, protein content, increased vigour, faster maturation, increased speed of seed emergence, improved nitrogen utilization efficiency, improved water use efficiency, improved oil content and /or quality, improved digestibility, faster ripening, improved flavor, improved starch content, more developed root system (improved root growth), improved stress tolerance (e.g. against drought, heat, salt, light, UV, water, cold), reduced ethylene (reduced production and/or inhibition of reception), tillering increase, increase in plant height, bigger leaf blade, less dead basal leaves, stronger tillers, greener leaf color, pigment content, photosynthetic activity, less input needed (such as fertilizers or water), less seeds needed, more productive tillers, earlier flowering, early grain maturity, less plant verse (lodging), increased shoot growth, enhanced plant vigor, increased plant stand and early and better germination.

Advantageous properties, obtained especially from treaded seeds, are e.g. improved germination and field establishment, better vigor, more homogeneous field establishment.

Advantageous properties, obtained especially from foliar and/or in-furrow application are e.g. improved plant growth and plant development, better growth, more tillers, greener leafes, largers leaves, more biomass, better roots, improved stress tolerance of the plants, more grain yield, more biomass harvested, improved quality of the harvest (content of fatty acids, metabolites, oil etc), more marketable products (e.g. improved size), improved process (e.g. longer shelf-life, better extraction of compounds), improved quality of seeds (for being seeded in the following seasons for seed production); or any other advantages familiar to a person skilled in the art.

It is therefore an object of the present invention to provide a method which solves the problems outlined above.

The present invention relates to plant-protecting active ingredients that are imidazole compounds of formula I according to the invention, in particular the individual imidazole compounds described in the above description as being preferred, and mixtures with increased efficacy and to a method of improving the health of plants by applying said compounds and mixtures to the plants or the locus thereof.

The action of the compounds of formula I goes beyond the known fungicidal action. The imidazole compounds of formula I according to the invention, in particular the individual imidazole compounds described in the above description as being preferred compounds exhibit plant health

The term plant health comprises various sorts of improvements of plants that are not connected to the control of harmful fungi.

The following non-limiting examples illustrate the above-described invention in more detail.

### Example 1: This example illustrates the preparation of 2-[5-(4-Bromo-phenyl)-2-chloro-4-methyl-imidazol-1-yl]-3,5-dichloro-pyridine (Compound No.I.s.035)

### a) Preparation of [1-(4-Bromo-phenyl)-meth-(E)-ylidene]-(3,5-dichloro-pyridin-2-yl)-amine

3,5-Dichloro-pyridin-2-ylamine (6 g) and 4-bromo-benzaldehyde (7.02 g) are dissolved in toluene (210 ml). Subsequently, the mixture is stirred for 3 days at reflux in a Dean-Stark apparatus. The reaction mixture is evaporated under reduced pressure, to obtain 12.213 g of [1-(4-bromo-phenyl)-meth-(E)-ylidene]-(3,5-dichloro-pyridin-2-yl)-amine. ¹H NMR (300Mhz, CDCl₃). 8.96ppm, 1H, s; 8.23ppm, 1H, d, J=2.25Hz; 7.81 ppm, 2H, d, J=8.46Hz; 7.72ppm, 1H, d, J=2.27Hz; 7.55ppm, 2H, d, J=8.45Hz.

### b) Preparation of 1-(Toluene-4-sulfonyl)-ethyl isocyanide

10 g of toluenesulfonylmethyl isocyanide and 2.31 g of benzyltriethylammonium chloride are dissolved in 100 ml of dichloromethane. 100 ml of a 30% sodium hydroxide solution in water are added and the mixture is cooled down to 0°C. 3.63 ml of methyl iodide are then added, and the reaction mixture is stirred vigorously for 2 h at 0°C. Water is then added, the phases are separated and the aqueous layer is extracted with dichloromethane. The combined organic phases are dried over sodium sulphate and filtrated; the solvent removed under reduced pressure. The black residue is extracted several times with cold diethyl ether and, after evaporation of the solvent, 4.2 g of 1-(toluene-4-sulfonyl)-ethyl isocyanide are isolated. An extra 1.8 g can be recovered again from the remaining residue by extracting it several times with t-butyl-methyl ether at room temperature. ¹H NMR (300Mhz, CDCl₃) 7.81 ppm, 2H, d, J=8.34Hz; 7.37ppm, 2H, d, J=8.04Hz; 4.52ppm, 1H, q, J=6.86Hz; 2.42ppm, 3H, s; 1.67ppm, 3H, d, J=6.81 Hz.

### c) Preparation of 2-[5-(4-Bromo-phenyl)-4-methyl-imidazol-1-yl]-3,5-dichloro-pyridine

0.9 g of [1-(4-bromo-phenyl)-meth-(E)-ylidene]-(3,5-dichloro-pyridin-2-yl)-amine is dissolved in 10 ml of N,N-dimethyl-formamide and 8 ml of 1,2-dimethoxy-ethane. 1.084 g of 1-(toluene-4-sulfonyl)-ethyl isocyanide and 0.754 g of anhydrous potassium carbonate are added, and the resulting reaction mixture is heated at 100°C overnight. After cooling down, the mixture is filtrated, the solvents evaporated, the crude material is adsorbed on Isolute^{®} HM-N and purified by chromatography column on silica gel, using a mixture of heptane / ethyl acetate 1:1 as eluent to obtain 0.509 g of 2-[5-(4-bromo-phenyl)-4-methyl-imidazol-1-yl]-3,5-dichloro-pyridine. ¹H NMR (300Mhz, CDCl₃) 8.31ppm, 1H, d, J=2.26Hz; 7.73ppm, 1H, d, J=2.23Hz; 7.61 ppm, 1H, s; 7.33ppm, 2H, d, J=8.47Hz; 6.88ppm, 2H, d, J=8.45Hz; 2.27ppm, 3H, s.

### d) Preparation of 2-[5-(4-Bromo-phenyl)-2-chloro-4-methyl-imidazol-1-yl]-3,5-dichloro-pyridine (Compound No.I.s.035)

A mixture of 0.251 g of 2-[5-(4-bromo-phenyl)-4-methyl-imidazol-1-yl]-3,5-dichloro-pyridine, 0.096 g of N-chlorosuccinimide and 2.11 ml of chloroform is heated for 90 minutes to 80 °C. Subsequently, the mixture is cooled to room temperature, Isolute^{®} HM-N is added to the reaction mixture and the chloroform is evaporated. The crude mixture is purified by chromatography column on silica gel, using a mixture of heptane / ethyl acetate 6:1 as eluent to obtain of 0.082 g of 2-[5-(4-bromo-phenyl)-2-chloro-4-methyl-imidazol-1-yl]-3,5-dichloro-pyridine. ¹H NMR (300Mhz, CDCl₃) 8.37ppm, 1H, d, J=2.25Hz; 7.76ppm, 1H, d, J=2.27Hz; 7.33ppm, 2H, d, J=8.45Hz; 6.93ppm, 2H, d, J=8.48Hz; 2.03ppm, 3H, s.

### Example 2: This example illustrates the preparation of 2-[5-(4-Bromo-phenyl)-2,4-dichloro-imidazol-1-yl]-3,5-dichloro-pyridine (Compound No.I.s.034)

### a) Preparation of 2-[5-(4-Bromo-phenyl)-imidazol-1-yl]-3,5-dichloro-pyridine

6 g of [1-(4-bromo-phenyl)-meth-(E)-ylidene]-(3,5-dichloro-pyridin-2-yl)-amine is dissolved in 125 ml of N,N-dimethyl-formamide and 55 ml of 1,2-dimethoxy-ethane. 5.38 g of Toluenesulfonylmethyl isocyanide and 5.02g of anhydrous potassium carbonate are added, and the resulting reaction mixture is heated at 100°C overnight. After cooling down, the mixture is filtrated, the solvents evaporated, the resulting solid is dissolved in 89 ml of methanol, and to this solution are added 4.428 g of anhydrous potassium carbonate. The resulting reaction mixture is heated at reflux of methanol for 4 h. After cooling down, the mixture is filtrated, the solvent evaporated, the resulting solid dissolved in 100 ml of dichloromethane and poured onto 100 ml of water. The organic phase is separated and washed with a sodium bicarbonate saturated aqueous solution (50 ml). The aqueous layer is separated and extracted with 50 ml of dichloromethane. The organic phases are combined, washed with 25 ml of brine, separated, dried over sodium sulphate, filtered and the solvent evaporated under reduced pressure. The crude material is adsorbed on Isolute^{®} HM-N and purified by chromatography column on silica gel, using a mixture of heptane / ethyl acetate 3:7 as eluent to obtain 2.110 g of 2-[5-(4-bromo-phenyl)-imidazol-1-yl]-3,5-dichloro-pyridine. ¹H NMR (300Mhz, CDCl₃) 8.37ppm, 1H, d, J=2.24Hz; 7.78ppm, 1H, d, J=2.26Hz; 7.68ppm, 1H, d, J=0.95Hz; 7.32ppm, 2H, d, J=8.58Hz; 7.21 ppm, 1H, d, J=0.96Hz; 6.90ppm, 2H, d, J=8.56Hz.

### b) Preparation of 2-[5-(4-Bromo-phenyl)-2,4-dichloro-imidazol-1-yl]-3,5-dichloro-pyridine (Compound No.I.s.034)

A mixture of 0.350 g of 2-[5-(4-bromo-phenyl)-imidazol-1-yl]-3,5-dichloro-pyridine, 0.253 g of N-chlorosuccinimide and 2.5 ml of chloroform is heated for 16 h to 80°C. Subsequently, the mixture is cooled to room temperature and the chloroform is evaporated. The crude mixture is dissolved in 5 ml of dichloromethane and poured onto 5 ml of a 1 N aqueous hydrochloric acid solution. The organic phase is separated and washed successively with a 1 N aqueous sodium hydroxide solution (5 ml) and 5 ml of brine. After separation, the organic phase is dried over sodium sulphate, filtered, and the solvent evaporated under reduced pressure. The crude material is adsorbed on Isolute^{®} HM-N and purified by chromatography column on silica gel, using a mixture of heptane / ethyl acetate 23:2 as eluent to obtain 0.224 g of 2-[5-(4-bromo-phenyl)-2,4-dichloro-imidazol-1-yl]-3,5-dichloro-pyridine. ¹H NMR (300Mhz, CDCl₃) 8.41 ppm, 1H, d, J=2.24Hz; 7.80ppm, 1H, d, J=2.23Hz; 7.36ppm, 2H, d, J=8.57Hz; 7.04ppm, 2H, d, J=8.50Hz.

Table 1 below illustrates examples of individual compounds of formula I according to the invention.

**Table 1: individual compounds of formula I according to the invention**

| **Compound No.** | **R¹** | **R²** | **R³** |
|---|---|---|---|
| 001 | F | phenyl | F |
| 002 | CH₃ | phenyl | F |
| 003 | F | phenyl | Cl |
| 004 | Cl | phenyl | Cl |
| 005 | CH₃ | phenyl | Cl |
| 006 | F | 3-fluorophenyl | F |
| 007 | CH₃ | 3-fluorophenyl | F |
| 008 | F | 3-fluorophenyl | Cl |
| 009 | Cl | 3-fluorophenyl | Cl |
| 010 | CH₃ | 3-fluorophenyl | Cl |
| 011 | F | 4-fluorophenyl | F |
| 012 | CH₃ | 4-fluorophenyl | F |
| 013 | F | 4-fluorophenyl | Cl |
| 014 | Cl | 4-fluorophenyl | Cl |
| 015 | CH₃ | 4-fluorophenyl | Cl |
| 016 | F | 3-chlorophenyl | F |
| 017 | CH₃ | 3-chlorophenyl | F |
| 018 | F | 3-chlorophenyl | Cl |
| 019 | Cl | 3-chlorophenyl | Cl |
| 020 | CH₃ | 3-chlorophenyl | Cl |
| 021 | F | 4-chlorophenyl | F |
| 022 | CH₃ | 4-chlorophenyl | F |
| 023 | F | 4-chlorophenyl | Cl |
| 024 | Cl | 4-chlorophenyl | Cl |
| 025 | CH₃ | 4-chlorophenyl | Cl |
| 026 | F | 3-bromophenyl | F |
| 027 | CH₃ | 3-bromophenyl | F |
| 028 | F | 3-bromophenyl | Cl |
| 029 | Cl | 3-bromophenyl | Cl |
| 030 | CH₃ | 3-bromophenyl | Cl |
| 031 | F | 4-bromophenyl | F |
| 032 | CH₃ | 4-bromophenyl | F |
| 033 | F | 4-bromophenyl | Cl |
| 034 | Cl | 4-bromophenyl | Cl |
| 035 | CH₃ | 4-bromophenyl | Cl |
| 036 | F | m-tolyl | F |
| 037 | CH₃ | m-tolyl | F |
| 038 | F | m-tolyl | Cl |
| 039 | Cl | m-tolyl | Cl |
| 040 | CH₃ | m-tolyl | Cl |
| 041 | F | p-tolyl | F |
| 042 | CH₃ | p-tolyl | F |
| 043 | F | p-tolyl | Cl |
| 044 | Cl | p-tolyl | Cl |
| 045 | CH₃ | p-tolyl | Cl |
| 046 | F | 3-trifluoromethylphenyl | F |
| 047 | CH₃ | 3-trifluoromethylphenyl | F |
| 048 | F | 3-trifluoromethylphenyl | Cl |
| 049 | Cl | 3-trifluoromethylphenyl | Cl |
| 050 | CH₃ | 3-trifluoromethylphenyl | Cl |
| 051 | F | 4-trifluoromethylphenyl | F |
| 052 | CH₃ | 4-trifluoromethylphenyl | F |
| 053 | F | 4-trifluoromethylphenyl | Cl |
| 054 | Cl | 4-trifluoromethylphenyl | Cl |
| 055 | CH₃ | 4-trifluoromethylphenyl | Cl |
| 056 | F | 3-methoxyphenyl | F |
| 057 | CH₃ | 3-methoxyphenyl | F |
| 058 | F | 3-methoxyphenyl | Cl |
| 059 | Cl | 3-methoxyphenyl | Cl |
| 060 | CH₃ | 3-methoxyphenyl | Cl |
| 061 | F | 4-methoxyphenyl | F |
| 062 | CH₃ | 4-methoxyphenyl | F |
| 063 | F | 4-methoxyphenyl | Cl |
| 064 | Cl | 4-methoxyphenyl | Cl |
| 065 | CH₃ | 4-methoxyphenyl | Cl |
| 066 | F | 3-trifluoromethoxyphenyl | F |
| 067 | CH₃ | 3-trifluoromethoxyphenyl | F |
| 068 | F | 3-trifluoromethoxyphenyl | Cl |
| 069 | Cl | 3-trifluoromethoxyphenyl | Cl |
| 070 | CH₃ | 3-trifluoromethoxyphenyl | Cl |
| 071 | F | 4-trifluoromethoxyphenyl | F |
| 072 | CH₃ | 4-trifluoromethoxyphenyl | F |
| 073 | F | 4-trifluoromethoxyphenyl | Cl |
| 074 | Cl | 4-trifluoromethoxyphenyl | Cl |
| 075 | CH₃ | 4-trifluoromethoxyphenyl | Cl |
| 076 | F | 3-cyanophenyl | F |
| 077 | CH₃ | 3-cyanophenyl | F |
| 078 | F | 3-cyanophenyl | Cl |
| 079 | Cl | 3-cyanophenyl | Cl |
| 080 | CH₃ | 3-cyanophenyl | Cl |
| 081 | F | 4-cyanophenyl | F |
| 082 | CH₃ | 4-cyanophenyl | F |
| 083 | F | 4-cyanophenyl | Cl |
| 084 | Cl | 4-cyanophenyl | Cl |
| 085 | CH₃ | 4-cyanophenyl | Cl |
| 086 | F | 3,4-difluorophenyl | F |
| 087 | CH₃ | 3,4-difluorophenyl | F |
| 088 | F | 3,4-difluorophenyl | Cl |
| 089 | Cl | 3,4-difluorophenyl | Cl |
| 090 | CH₃ | 3,4-difluorophenyl | Cl |
| 091 | F | 3,4-dichlorophenyl | F |
| 092 | CH₃ | 3,4-dichlorophenyl | F |
| 093 | F | 3,4-dichlorophenyl | Cl |
| 094 | Cl | 3,4-dichlorophenyl | Cl |
| 095 | CH₃ | 3,4-dichlorophenyl | Cl |
| 096 | F | 3,4-dimethylphenyl | F |
| 097 | CH₃ | 3,4-dimethylphenyl | F |
| 098 | F | 3,4-dimethylphenyl | Cl |
| 099 | Cl | 3,4-dimethylphenyl | Cl |
| 100 | CH₃ | 3,4-dimethylphenyl | Cl |
| 101 | F | 3,4-dimethoxyphenyl | F |
| 102 | CH₃ | 3,4-dimethoxyphenyl | F |
| 103 | F | 3,4-dimethoxyphenyl | Cl |
| 104 | Cl | 3,4-dimethoxyphenyl | Cl |
| 105 | CH₃ | 3,4-dimethoxyphenyl | Cl |
| 106 | F | 3-chloro-4-fluorophenyl | F |
| 107 | CH₃ | 3-chloro-4-fluorophenyl | F |
| 108 | F | 3-chloro-4-fluorophenyl | Cl |
| 109 | Cl | 3-chloro-4-fluorophenyl | Cl |
| 110 | CH₃ | 3-chloro-4-fluorophenyl | Cl |
| 111 | F | 3-chloro-4-methylphenyl | F |
| 112 | CH₃ | 3-chloro-4-methylphenyl | F |
| 113 | F | 3-chloro-4-methylphenyl | Cl |
| 114 | Cl | 3-chloro-4-methylphenyl | Cl |
| 115 | CH₃ | 3-chloro-4-methylphenyl | Cl |
| 116 | F | 3-chloro-4-methoxyphenyl | F |
| 117 | CH₃ | 3-chloro-4-methoxyphenyl | F |
| 118 | F | 3-chloro-4-methoxyphenyl | Cl |
| 119 | Cl | 3-chloro-4-methoxyphenyl | Cl |
| 120 | CH₃ | 3-chloro-4-methoxyphenyl | Cl |
| 121 | F | 4-chloro-3-fluorophenyl | F |
| 122 | CH₃ | 4-chloro-3-fluorophenyl | F |
| 123 | F | 4-chloro-3-fluorophenyl | Cl |
| 124 | Cl | 4-chloro-3-fluorophenyl | Cl |
| 125 | CH₃ | 4-chloro-3-fluorophenyl | Cl |
| 126 | F | 4-chloro-3-methylphenyl | F |
| 127 | CH₃ | 4-chloro-3-methylphenyl | F |
| 128 | F | 4-chloro-3-methylphenyl | Cl |
| 129 | Cl | 4-chloro-3-methylphenyl | Cl |
| 130 | CH₃ | 4-chloro-3-methylphenyl | Cl |
| 131 | F | 4-chloro-3-methoxyphenyl | F |
| 132 | CH₃ | 4-chloro-3-methoxyphenyl | F |
| 133 | F | 4-chloro-3-methoxyphenyl | Cl |
| 134 | Cl | 4-chloro-3-methoxyphenyl | Cl |
| 135 | CH₃ | 4-chloro-3-methoxyphenyl | Cl |
| 136 | F | 3-fluoro-4-methoxyphenyl | F |
| 137 | CH₃ | 3-fluoro-4-methoxyphenyl | F |
| 138 | F | 3-fluoro-4-methoxyphenyl | Cl |
| 139 | Cl | 3-fluoro-4-methoxyphenyl | Cl |
| 140 | CH₃ | 3-fluoro-4-methoxyphenyl | Cl |
| 141 | F | 3-fluoro-4-methylphenyl | F |
| 142 | CH₃ | 3-fluoro-4-methylphenyl | F |
| 143 | F | 3-fluoro-4-methylphenyl | Cl |
| 144 | Cl | 3-fluoro-4-methylphenyl | Cl |
| 145 | CH₃ | 3-fluoro-4-methylphenyl | Cl |
| 146 | F | 4-fluoro-3-methoxyphenyl | F |
| 147 | CH₃ | 4-fluoro-3-methoxyphenyl | F |
| 148 | F | 4-fluoro-3-methoxyphenyl | Cl |
| 149 | Cl | 4-fluoro-3-methoxyphenyl | Cl |
| 150 | CH₃ | 4-fluoro-3-methoxyphenyl | Cl |
| 151 | F | 4-fluoro-3-methylphenyl | F |
| 152 | CH₃ | 4-fluoro-3-methylphenyl | F |
| 153 | F | 4-fluoro-3-methylphenyl | Cl |
| 154 | Cl | 4-fluoro-3-methylphenyl | Cl |
| 155 | CH₃ | 4-fluoro-3-methylphenyl | Cl |
| 156 | F | 3-methoxy-4-methylphenyl | F |
| 157 | CH₃ | 3-methoxy-4-methylphenyl | F |
| 158 | F | 3-methoxy-4-methylphenyl | Cl |
| 159 | Cl | 3-methoxy-4-methylphenyl | Cl |
| 160 | CH₃ | 3-methoxy-4-methylphenyl | Cl |
| 161 | F | 4-methoxy-3-methylphenyl | F |
| 162 | CH₃ | 4-methoxy-3-methylphenyl | F |
| 163 | F | 4-methoxy-3-methylphenyl | Cl |
| 164 | Cl | 4-methoxy-3-methylphenyl | Cl |
| 165 | CH₃ | 4-methoxy-3-methylphenyl | Cl |
| 166 | F | naphth-2-yl | F |
| 167 | CH₃ | naphth-2-yl | F |
| 168 | F | naphth-2-yl | Cl |
| 169 | Cl | naphth-2-yl | Cl |
| 170 | CH₃ | naphth-2-yl | Cl |
| 171 | F | pyridin-2-yl | F |
| 172 | CH₃ | pyridin-2-yl | F |
| 173 | F | pyridin-2-yl | Cl |
| 174 | Cl | pyridin-2-yl | Cl |
| 175 | CH₃ | pyridin-2-yl | Cl |
| 176 | F | pyridin-3-yl | F |
| 177 | CH₃ | pyridin-3-yl | F |
| 178 | F | pyridin-3-yl | Cl |
| 179 | Cl | pyridin-3-yl | Cl |
| 180 | CH₃ | pyridin-3-yl | Cl |
| 181 | F | pyridin-4-yl | F |
| 182 | CH₃ | pyridin-4-yl | F |
| 183 | F | pyridin-4-yl | Cl |
| 184 | Cl | pyridin-4-yl | Cl |
| 185 | CH₃ | pyridin-4-yl | Cl |
| 186 | F | 6-chloropyridin-2-yl | F |
| 187 | CH₃ | 6-chloropyridin-2-yl | F |
| 188 | F | 6-chloropyridin-2-yl | Cl |
| 189 | Cl | 6-chloropyridin-2-yl | Cl |
| 190 | CH₃ | 6-chloropyridin-2-yl | Cl |
| 191 | F | 6-fluoropyridin-2-yl | F |
| 192 | CH₃ | 6-fluoropyridin-2-yl | F |
| 193 | F | 6-fluoropyridin-2-yl | Cl |
| 194 | Cl | 6-fluoropyridin-2-yl | Cl |
| 195 | CH₃ | 6-fluoropyridin-2-yl | Cl |
| 196 | F | 6-methoxypyridin-2-yl | F |
| 197 | CH₃ | 6-methoxypyridin-2-yl | F |
| 198 | F | 6-methoxypyridin-2-yl | Cl |
| 199 | Cl | 6-methoxypyridin-2-yl | Cl |
| 200 | CH₃ | 6-methoxypyridin-2-yl | Cl |
| 201 | F | 6-methylpyridin-2-yl | F |
| 202 | CH₃ | 6-methylpyridin-2-yl | F |
| 203 | F | 6-methylpyridin-2-yl | Cl |
| 204 | Cl | 6-methylpyridin-2-yl | Cl |
| 205 | CH₃ | 6-methylpyridin-2-yl | Cl |
| 206 | F | 6-chloropyridin-3-yl | F |
| 207 | CH₃ | 6-chloropyridin-3-yl | F |
| 208 | F | 6-chloropyridin-3-yl | Cl |
| 209 | Cl | 6-chloropyridin-3-yl | Cl |
| 210 | CH₃ | 6-chloropyridin-3-yl | Cl |
| 211 | F | 6-fluoropyridin-3-yl | F |
| 212 | CH₃ | 6-fluoropyridin-3-yl | F |
| 213 | F | 6-fluoropyridin-3-yl | Cl |
| 214 | Cl | 6-fluoropyridin-3-yl | Cl |
| 215 | CH₃ | 6-fluoropyridin-3-yl | Cl |
| 216 | F | 6-methoxypyridin-3-yl | F |
| 217 | CH₃ | 6-methoxypyridin-3-yl | F |
| 218 | F | 6-methoxypyridin-3-yl | Cl |
| 219 | Cl | 6-methoxypyridin-3-yl | Cl |
| 220 | CH₃ | 6-methoxypyridin-3-yl | Cl |
| 221 | F | 6-methylpyridin-3-yl | F |
| 222 | CH₃ | 6-methylpyridin-3-yl | F |
| 223 | F | 6-methylpyridin-3-yl | Cl |
| 224 | Cl | 6-methylpyridin-3-yl | Cl |
| 225 | CH₃ | 6-methylpyridin-3-yl | Cl |
| 226 | F | 2-chloropyridin-4-yl | F |
| 227 | CH₃ | 2-chloropyridin-4-yl | F |
| 228 | F | 2-chloropyridin-4-yl | Cl |
| 229 | Cl | 2-chloropyridin-4-yl | Cl |
| 230 | CH₃ | 2-chloropyridin-4-yl | Cl |
| 231 | F | 2-fluoropyridin-4-yl | F |
| 232 | CH₃ | 2-fluoropyridin-4-yl | F |
| 233 | F | 2-fluoropyridin-4-yl | Cl |
| 234 | Cl | 2-fluoropyridin-4-yl | Cl |
| 235 | CH₃ | 2-fluoropyridin-4-yl | Cl |
| 236 | F | 2-methoxypyridin-4-yl | F |
| 237 | CH₃ | 2-methoxypyridin-4-yl | F |
| 238 | F | 2-methoxypyridin-4-yl | Cl |
| 239 | Cl | 2-methoxypyridin-4-yl | Cl |
| 240 | CH₃ | 2-methoxypyridin-4-yl | Cl |
| 241 | F | 2-methylpyridin-4-yl | F |
| 242 | CH₃ | 2-methylpyridin-4-yl | F |
| 243 | F | 2-methylpyridin-4-yl | Cl |
| 244 | Cl | 2-methylpyridin-4-yl | Cl |
| 245 | CH₃ | 2-methylpyridin-4-yl | Cl |
| 246 | F | 5-chlorothiophen-2-yl | F |
| 247 | CH₃ | 5-chlorothiophen-2-yl | F |
| 248 | F | 5-chlorothiophen-2-yl | Cl |
| 249 | Cl | 5-chlorothiophen-2-yl | Cl |
| 250 | CH₃ | 5-chlorothiophen-2-yl | Cl |
| 251 | F | 5-bromothiophen-2-yl | F |
| 252 | CH₃ | 5-bromothiophen-2-yl | F |
| 253 | F | 5-bromothiophen-2-yl | Cl |
| 254 | Cl | 5-bromothiophen-2-yl | Cl |
| 255 | CH₃ | 5-bromothiophen-2-yl | Cl |
| 256 | F | 5-methoxythiophen-2-yl | F |
| 257 | CH₃ | 5-methoxythiophen-2-yl | F |
| 258 | F | 5-methoxythiophen-2-yl | Cl |
| 259 | Cl | 5-methoxythiophen-2-yl | Cl |
| 260 | CH₃ | 5-methoxythiophen-2-yl | Cl |
| 261 | F | quinolin-2-yl | F |
| 262 | CH₃ | quinolin-2-yl | F |
| 263 | F | quinolin-2-yl | Cl |
| 264 | Cl | quinolin-2-yl | Cl |
| 265 | CH₃ | quinolin-2-yl | Cl |
| 266 | F | quinolin-3-yl | F |
| 267 | CH₃ | quinolin-3-yl | F |
| 268 | F | quinolin-3-yl | Cl |
| 269 | Cl | quinolin-3-yl | Cl |
| 270 | CH₃ | quinolin-3-yl | Cl |
| 271 | F | 4-methoxyquinolin-2-yl | F |
| 272 | CH₃ | 4-methoxyquinolin-2-yl | F |
| 273 | F | 4-methoxyquinolin-2-yl | Cl |
| 274 | Cl | 4-methoxyquinolin-2-yl | Cl |
| 275 | CH₃ | 4-methoxyquinolin-2-yl | Cl |
| 276 | F | 4-methylquinolin-2-yl | F |
| 277 | CH₃ | 4-methylquinolin-2-yl | F |
| 278 | F | 4-methylquinolin-2-yl | Cl |
| 279 | Cl | 4-methylquinolin-2-yl | Cl |
| 280 | CH₃ | 4-methylquinolin-2-yl | Cl |

where
a) 110 compounds of formula (I.a): 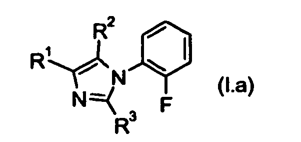 wherein R¹, R² and R³ are as defined in Table 1 as compounds 171 to 280.
b) 110 compounds of formula (I.b): 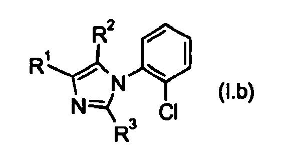 wherein R¹, R² and R³ are as defined in Table 1 as compounds 171 to 280.
c) 110 compounds of formula (I.c): 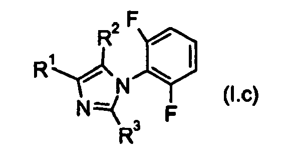 wherein R¹, R² and R³ are as defined in Table 1 as compounds 171 to 280.
d) 110 compounds of formula (I.d): 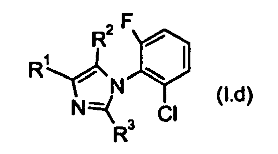 wherein R¹, R² and R³ are as defined in Table 1 as compounds 171 to 280.
e) 110 compounds of formula (I.e): 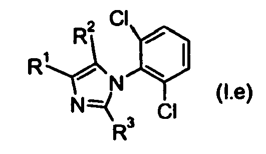 wherein R¹, R² and R³ are as defined in Table 1 as compounds 171 to 280.
f) 110 compounds of formula (I.f): 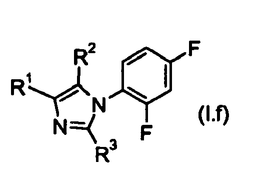 wherein R¹, R² and R³ are as defined in Table 1 as compounds 171 to 280.
g) 110 compounds of formula (I.g): 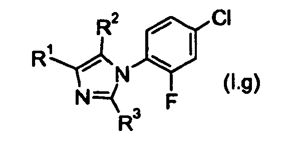 wherein R¹, R² and R³ are as defined in Table 1 as compounds 171 to 280.
h) 110 compounds of formula (I.h): 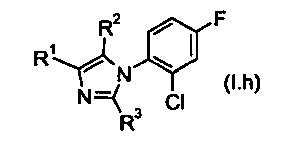 wherein R¹, R² and R³ are as defined in Table 1 as compounds 171 to 280.
i) 110 compounds of formula (I.i): 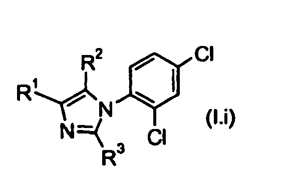 wherein R¹, R² and R³ are as defined in Table 1 as compounds 171 to 280.
j) 110 compounds of formula (I.j): 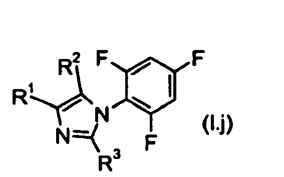 wherein R¹, R² and R³ are as defined in Table 1 as compounds 171 to 280.
k) 110 compounds of formula (I.k): 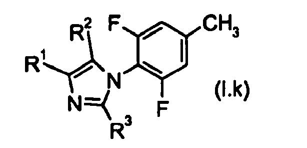 wherein R¹, R² and R³ are as defined in Table 1 as compounds 171 to 280.
l) 110 compounds of formula (I.I): 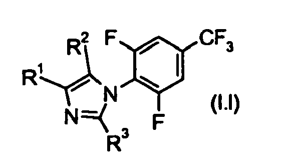 wherein R¹, R² and R³ are as defined in Table 1 as compounds 171 to 280.
m) 110 compounds of formula (I.m): 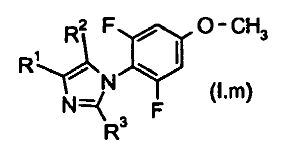 wherein R¹, R² and R³ are as defined in Table 1 as compounds 171 to 280.
n) 110 compounds of formula (I.n): 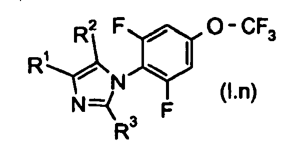 wherein R¹, R² and R³ are as defined in Table 1 as compounds 171 to 280.
o) 110 compounds of formula (I.o): 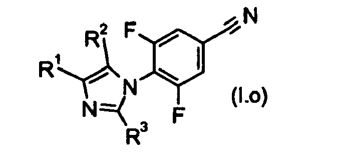 wherein R¹, R² and R³ are as defined in Table 1 as compounds 171 to 280.
p) 280 compounds of formula (I.p): 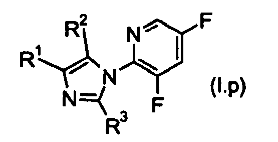 wherein R¹, R² and R³ are as defined in Table 1.
q) 280 compounds of formula (I.q): 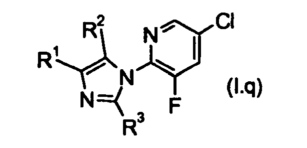 wherein R¹, R² and R³ are as defined in Table 1.
r) 280 compounds of formula (I.r): 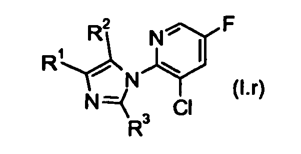 wherein R¹, R² and R³ are as defined in Table 1.
s) 280 compounds of formula (I.s): 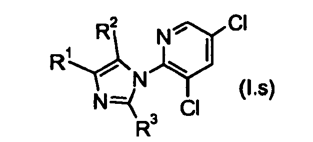 wherein R¹, R² and R³ are as defined in Table 1.
t) 280 compounds of formula (I.t): 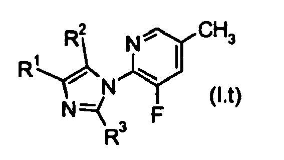 wherein R¹, R² and R³ are as defined in Table 1.
u) 280 compounds of formula (I.u): 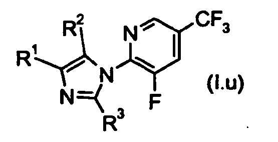 wherein R¹, R² and R³ are as defined in Table 1.
v) 280 compounds of formula (I.v): 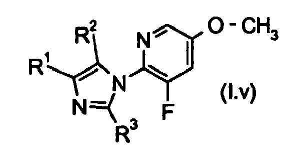 wherein R¹, R² and R³ are as defined in Table 1.
w) 280 compounds of formula (I.w): 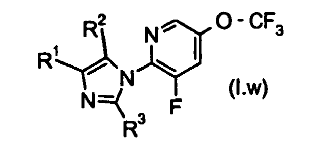 wherein R¹, R² and R³ are as defined in Table 1.
x) 280 compounds of formula (I.x): 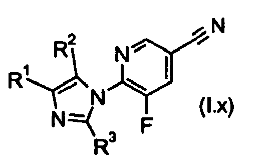 wherein R¹, R² and R³ are as defined in Table 1.
y) 280 compounds of formula (I.y): 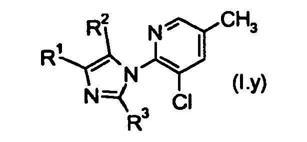 wherein R¹, R² and R³ are as defined in Table 1.
z) 280 compounds of formula (I.z): 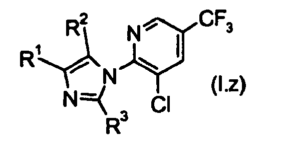 wherein R¹, R² and R³ are as defined in Table 1.
aa) 280 compounds of formula (I.aa): 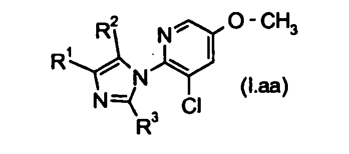 wherein R¹, R² and R³ are as defined in Table 1.
ab) 280 compounds of formula (Lab): 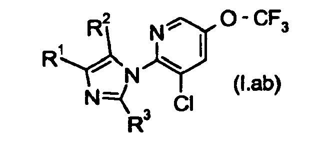 wherein R¹, R² and R³ are as defined in Table 1.
ac) 280 compounds of formula (I.ac): 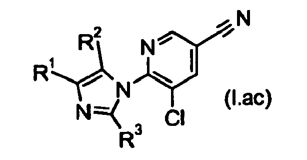 wherein R¹, R² and R³ are as defined in Table 1.

Throughout this description, temperatures are given in degrees Celsius, "m.p." means melting point, "NMR" means nuclear magnetic resonance spectrum; and "%" is percent by weight, unless corresponding concentrations are indicated in other units.

The following abbreviations are used throughout this description:

| | | | |
|---|---|---|---|
| m.p. = | melting point | br = | broad |
| s = | singlet | dd = | doublet of doublets |
| d = | doublet | dt = | doublet of triplets |
| t = | triplet | q = | quartet |
| m = | multiplet | ppm = | parts per million |

Table 2 shows selected NMR data, all with CDCl₃ as the solvent (unless otherwise stated, no attempt is made to list all characterising data in all cases) for compounds of Table 1.

**Table 2: Melting point and selected NMR data for compounds of Table 1**

| Compound Number | ¹H-NMR data (ppm/number of H's/multiplicity) |
|---|---|
| I.s.034 | 8.41 ppm, 1H, d, J=2.24Hz; 7.80ppm, 1H, d, J=2.23Hz; 7.36ppm, 2H, d, J=8.57Hz; 7.04ppm, 2H, d, J=8.50Hz. |
| I.s.035 | 8.37ppm, 1H, d, J=2.25Hz; 7.76ppm, 1H, d, J=2.27Hz; 7.33ppm, 2H, d, J=8.45Hz; 6.93ppm, 2H, d, J=8.48Hz; 2.03ppm, 3H, s. |
| I.s.210 | 8.38ppm, 1H, d, J=2.21 Hz; 8.11 ppm, 1H, d, J=2.14Hz; 7.79ppm, 1H, d, J=2.21 Hz; 7.39ppm, 1H, dd, J=2.49 and 8.19Hz; 7.20ppm, 1H, d, J=8.20Hz; 2.23ppm, 3H, s. |
| I.s.269 | 8.62ppm, 1H, d, J=2.23Hz; 8.40ppm, 1H, d, J=2.22Hz; 8.11 ppm, 1H, d, J=2.03Hz; 7.98ppm, 1H, d, J=8.55Hz; 7.78ppm, 1H, d, J=2.27Hz; 7.72ppm, 1H, d, J=8.20Hz; 7.68ppm, 1H, dt, J=1.39 and 8.41 Hz; 7.51 ppm, 1H, dt, J=0.94 and 7.56Hz. |
| I.s.270 | 8.60ppm, 1H, d, J=2.20Hz; 8.36ppm, 1H, d, J=2.25Hz; 7.98ppm, 1H, d, J=8.45Hz; 7.94ppm, 1H, d, J=2.03Hz; 7.74ppm, 1H, d, J=2.33Hz; 7.70ppm, 1H, d, J=8.09Hz; 7.66ppm, 1H, dt, J=1.39 and 7.19Hz; 7.50ppm, 1H, dt, J=1.02 and 8.01 Hz; 2.30ppm, 3H, s. |

The compounds according to the present invention can be prepared according to the above-mentioned reaction schemes, in which, unless otherwise stated, the definition of each variable is as defined above for a compound of formula (I).

### Biological examples

### Alternaria solani / tomato / preventive (Action against Alternaria on tomato)

4 weeks old tomato plants cv. Roter Gnom are treated with the formulated test compound in a spray chamber. Two days after application tomato plants are inoculated by spraying a spore suspension on the test plants. After an incubation period of 4 days at 22 °C / 18 °C and 95% r. h. in a greenhouse the disease incidence is assessed.

Compounds I.s.034, I.s.035, I.s.210, I.s.269 and I.s.270 at 200 ppm inhibit fungal infestation in this test to at least 80 %, while under the same conditions untreated control plants are infected by the phytopathogenic fungi to over 80 %.

### Botrytis cinerea / tomato / preventive (Action against Botrytis on tomato)

4 weeks old tomato plants cv. Roter Gnom are treated with the formulated test compound in a spray chamber. Two days after application tomato plants are inoculated by spraying a spore suspension on the test plants. After an incubation period of 3 days at 20 °C and 95% r. h. in a greenhouse the disease incidence is assessed.

Compounds I.s.034 at 200 ppm inhibit fungal infestation in this test to at least 80 %, while under the same conditions untreated control plants are infected by the phytopathogenic fungi to over 80 %.

### Puccinia recondita /wheat / preventive (Action against brown rust on wheat)

1 week old wheat plants cv. Arina are treated with the formulated test compound in a spray chamber. One day after application wheat plants are inoculated by spraying a spore suspension (1 x 105 uredospores/ml) on the test plants. After an incubation period of 1 day at 20 °C and 95% r. h. plants are kept for 10 days 20 °C / 18 °C (day/night) and 60% r.h. in a greenhouse. The disease incidence is assessed 11 days after inoculation.

Compounds I.s.035 at 200 ppm inhibit fungal infestation in this test to at least 80 %, while under the same conditions untreated control plants are infected by the phytopathogenic fungi to over 80 %.

### Magnaporthe grisea (Pyricularia oryzae) / rice / preventive (Action against rice blast)

3 weeks old rice plants cv. Koshihikari are treated with the formulated test compound in a spray chamber. Two days after application rice plants are inoculated by spraying a spore suspension (1 x 10⁵ conidia/ml) on the test plants. After an incubation period of 6 days at 25°C and 95% r. h. the disease incidence is assessed.

### Pyrenophora teres (Helminthosporium teres) / barley / preventive (Action against net blotch on barley)

1-week-old barley plants cv. Regina are treated with the formulated test compound in a spray chamber. Two days after application barley plants are inoculated by spraying a spore suspension (2.6 x 10⁴ conidia/ml) on the test plants. After an incubation period of 4 days at 20 °C and 95% r. h. the disease incidence is assessed.

### Septoria tritici/wheat / preventive (Action against Septoria leaf spot on wheat)

2 weeks old wheat plants cv. Riband are treated with the formulated test compound in a spray chamber. One day after application wheat plants are inoculated by spraying a spore suspension (10⁶ conidia/ml) on the test plants. After an incubation period of 1 day at 22 °C / 21 °C and 95% r. h. plants are kept at 22 °C / 21°C and 70% r.h. in a greenhouse. The disease incidence is assessed 16 - 18 days after inoculation.

Compounds I.s.034, I.s.035, I.s.210, I.s.269 and I.s.270 at 200 ppm inhibits fungal infestation in this test to at least 80 %, while under the same conditions untreated control plants are infected by the phytopathogenic fungi to over 80 %.

### Uncinula necator / grape / Preventive (Action against powdery mildew on grape)

5 weeks old grape seedlings cv. Gutedel are treated with the formulated test compound in a spray chamber. One day after application grape plants are inoculated by shaking plants infected with grape powdery mildew above the test plants. After an incubation period of 7 days at 24 °C / 22 °C and 70% r. h. under a light regime of 14/10 h (light/dark) the disease incidence is assessed.

Compounds I.s.034, I.s.035, I.s.210, I.s.269 and I.s.270 at 200 ppm inhibit fungal infestation in this test to at least 80 %, while under the same conditions untreated control plants are infected by the phytopathogenic fungi to over 80 %.

## Claims

1. A compound of formula I: 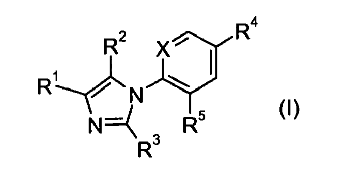 wherein
R¹ is halogen, C₁-C₄alkyl or C₁-C₄haloalkyl;
R² is an optionally substituted aryl or heteroaryl;
R³ is halogen;
R⁴ is hydrogen, halogen, C₁-C₄alkyl, C₁-C₄haloalkyl, C₁-C₄alkoxy, C₁-C₄haloalkoxy or cyano; R⁵ is halogen;
X is N or C(R); and
R is hydrogen, halogen, C₁-C₄alkyl, C₁-C₄haloalkyl, C₁-C₄alkoxy, C₁-C₄haloalkoxy or cyano; or an agrochemically usable salt form thereof;
provided that
when X is C(R), R² cannot be an optionally substituted aryl.

2. The compound according to claim 1 wherein R¹ is halogen, C₁-C₃alkyl or C₁-C₃haloalkyl.

3. The compound according to either claims 1 or 2 wherein R² is an optionally substituted phenyl, naphthyl, pyridyl, thienyl or quinolyl.

4. The compound according to any one of claims 1 to 3 wherein R³ is fluoro, chloro, bromo or iodo.

5. The compound according to any one of claims 1 to 4 wherein R⁴ is hydrogen, halogen, C₁-C₃alkyl, C₁-C₃haloalkyl, C₁-C₃alkoxy, C₁-C₃haloalkoxy or cyano.

6. The compound according to any one of claims 1 to 5 wherein R⁵ is fluoro, chloro, bromo or iodo.

7. The compound according to any one of claims 1 to 6 wherein X is N, C(H), C(halogen), C(C₁-C₄alkyl), C(C₁-C₄haloalkyl), C(C₁-C₄alkoxy) or C(C₁-C₄haloalkoxy).

8. The compound according to any one of claims 1 to 7 wherein
R¹ is fluoro, chloro, bromo, iodo, C₁-C₂alkyl or C₁-C₂haloalkyl;
R² is an optionally substituted phenyl, pyridyl, thienyl or quinolyl;
R³ is fluoro, chloro or bromo;
R⁴ is hydrogen, fluoro, chloro, bromo, C₁-C₃alkyl, C₁-C₃haloalkyl, C₁-C₃alkoxy, C₁-C₃haloalkoxy or cyano;
R⁵ is fluoro, chloro or bromo; and
X is N, C(H), C(Cl), C(F), C(Br), C(I), C(C₁-C₃alkyl), C(C₁-C₃haloalkyl), C(C₁-C₃alkoxy) or C(C₁-C₃haloalkoxy).

9. The compound according to any one of claims 1 to 8 wherein
R¹ is fluoro, chloro, bromo, methyl or ethyl;
R² is phenyl, 3-fluorophenyl, 4-fluorophenyl, 3-chlorophenyl, 4-chlorophenyl, 3-bromophenyl, 4-bromophenyl, m-tolyl, p-tolyl, 3-trifluoromethylphenyl, 4-trifluorometlaylphenyl, 3-methoxyphenyl, 4-methoxyphenyl, 3-trifluoromethoxyphenyl, 4-trifluoromethoxyphenyl, 3-cyanophenyl, 4-cyanophenyl, 3,4-difluorophenyl, 3,4-dichlorophenyl, 3,4-dimethylphenyl, 3,4-dimethoxyphenyl, 3-chloro-4-fluorophenyl, 3-chloro-4-methylphenyl, 3-chloro-4-methoxyphenyl, 4-chloro-3-fluorophenyl, 4-chloro-3-methylphenyl, 4-chloro-3-methoxyphenyl, 3-fluoro-4-methoxyphenyl, 3-fluoro-4-methylphenyl, 4-fluoro-3-methoxyphenyl, 4-fluoro-3-methylphenyl, 3-methoxy-4-methylphenyl, 4-methoxy-3-methylphenyl, , pyridin-2-yl, pyridin-3-yl, pyridin-4-yl, 6-chloropyridin-2-yl, 6-fluoropyridin-2-yl, 6-methoxypyridin-2-yl, 6-methylpyridin-2-yl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, 5-chlorothiophen-2-yl, 5-bromothiophen-2-yl, 5-methoxythiophen-2-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl, C₁-C₂alkoxy, C₁-C₂haloalkoxy or cyano;
R⁵ is fluoro or chloro; and
X is N, C(H), C(Cl), C(F), C(Br), C(C,-C₂alkyl), C(C₁-C₂haloalkyl), C(C₁-C₂alkoxy) or C(C₁-C₂haloalkoxy).

10. The compound according to any one of claims 1 to 9 wherein
R¹ is fluoro, chloro, methyl or ethyl;
R² is 4-fluorophenyl, 4-chlorophenyl, 4-bromophenyl, 6-chloropyridin-3-yl, 6-fluoropyridin-3-yl, 6-methoxypyridin-3-yl, 6-methylpyridin-3-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl or C₁-C₂alkoxy;
R⁵ is fluoro or chloro; and
X is N, C(H), C(Cl) or C(F).

11. The compound according to any one of claims 1 to 10 wherein
R¹ is chloro or methyl;
R² is 4-bromophenyl, 6-chloropyridin-3-yl or quinolin-3-yl;
R³ is chloro;
R⁴ is chloro;
R⁵ is chloro; and
X is N.

12. A compound according to claim 1 selected from
2-Chloro-5-[2,5-dichloro-3-(2,4,6-trifluoro-phenyl)-3H-imidazol-4-yl]-pyridine;
2-Chloro-5-[2-chloro-5-methyl-3-(2,4,6-trifluoro-phenyl)-3H-imidazol-4-yl]-pyridine;
5-[2-Chloro-5-methyl-3-(2,4,6-trifluoro-phenyl)-3H-imidazol-4-yl]-2-methoxy-pyridine;
3-[2-Chloro-5-methyl-3-(2,4,6-trifluoro-phenyl)-3H-imidazol-4-yl]-quinoline;
3-[2,5-Dichloro-3-(2,4,6-trifluoro-phenyl)-3H-imidazol-4-yl]-quinoline;
3-[2-Chloro-3-(2,6-difluoro-4-methoxy-phenyl)-5-methyl-3H-imidazol-4-yl]-quinoline;
3-[2,5-Dichloro-3-(2,6-difluoro-4-methoxy-phenyl)-3H-imidazol-4-yl]-quinoline;
2-[2-Chloro-5-methyl-3-(2,4,6-trifluoro-phenyl)-3H-imidazol-4-yl]-quinoline;
2-[2-Chloro-5-methyl-3-(2,4,6-trifluoro-phenyl)-3H-imidazol-4-yl]-4-methoxy-quinoline;
2-[2-Chloro-3-(2,6-difluoro-4-methoxy-phenyl)-5-methyl-3H-imidazol-4-yl]-4-methoxy-quinoline;
3,5-Dichloro-2-[2,4-dichloro-5-(6-chloro-pyridin-3-yl)-imidazol-1-yl]-pyridine;
3,5-Dichloro-2-[2-chloro-5-(6-chloro-pyridin-3-yl)-4-methyl-imidazol-1-yl]-pyridine;
5-[2-Chloro-3-(2,6-difluoro-4-methoxy-phenyl)-5-methyl-3H-imidazol-4-yl]-2-methyl-pyridine; and
3,5-Dichloro-2-[2-chloro-5-(4-methoxy-phenyl)-4-methyl-imidazol-1-yl]-pyridine.

13. A fungicidal composition for controlling or protecting against phytopathogenic microorganisms, comprising as active ingredient at least one compound as defined in any one of claims 1 to 12, in free form or in agrochemically usable salt form, and at least one adjuvant.

14. The composition according to claim 16, which comprises at least one additional fungicidally active compound, preferably selected from the group consisting of azoles, pyrimidinyl carbinoles, 2-amino-pyrimidines, morpholines, anilinopyrimidines, pyrroles, phenylamides, benzimidazoles, dicarboximides, carboxamides, strobilurines, dithiocarbamates, N-halomethylthiotetrahydrophthalimides, copper-compounds, nitrophenols, organo-phosphorus-derivatives, pyridazines, triazolopyrimidines, carboxamides or benzamides.

15. The use of a compound as defined in any one of claims 1 to 12 for controlling or preventing infestation of plants, harvested food crops, seeds or non-living materials by phytopathogenic microorganisms.

16. A method of controlling or preventing an infestation of crop plants, harvested food crops or non-living materials by phytopathogenic or spoilage microorganisms or organisms potentially harmful to man, which comprises the application of a compound as defined in any one of claims 1 to 12, as active ingredient to the plants, to parts of the plants or to the locus thereof, to seeds or to any part of the non-living materials.

17. The method according to claim 16, wherein the phytopathogenic microorganisms are fungal organisms.

## Patentansprüche

1. Verbindung der Formel I: 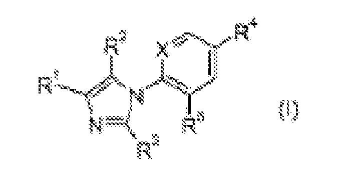 in der
R¹ Halogen, C₁-C₄-Alkyl oder C₁-C₄-Halogenalkyl bedeutet;
R² ein gegebenenfalls substituiertes Aryl oder Heteroaryl bedeutet;
R³ Halogen bedeutet;
R⁴ Wasserstoff, Halogen, C₁-C₄-Alkyl, C₁-C₄-Halogenalkyl, C₁-C₄-Alkoxy, C₁-C₄-Halogenalkoxy oder Cyano bedeutet;
R⁵ Halogen bedeutet;
X N oder C(R) bedeutet; und
R Wasserstoff, Halogen, C₁-C₄-Alkyl, C₁-C₄-Halogenalkyl, C₁-C₄-Alkoxy, C₁-C₄-Halogenalkoxy oder Cyano bedeutet;
oder eine agrarchemisch verwendbare Salzform davon;
mit der Maßgabe, dass,
wenn X C(R) bedeutet, R² nicht ein gegebenenfalls substituiertes Aryl bedeuten kann.

2. Verbindung nach Anspruch 1, wobei R¹ Halogen, C₁-C₃-Alkyl oder C₁-C₃-Halogenalkyl bedeutet.

3. Verbindung nach einem der Ansprüche 1 oder 2, wobei R² ein gegebenenfalls substituiertes Phenyl, Naphthyl, Pyridyl, Thienyl oder Chinolyl bedeutet.

4. Verbindung nach einem der Ansprüche 1 bis 3, wobei R³ Fluor, Chlor, Brom oder Iod bedeutet.

5. Verbindung nach einem der Ansprüche 1 bis 4, wobei R⁴ Wasserstoff, Halogen, C₁-C₃-Alkyl, C₁-C₃-Halogenalkyl, C₁-C₃-Alkoxy, C₁-C₃-Halogenalkoxy oder Cyano bedeutet.

6. Verbindung nach einem der Ansprüche 1 bis 5, wobei R⁵ Fluor, Chlor, Brom oder Iod bedeutet.

7. Verbindung nach einem der Ansprüche 1 bis 6, wobei X N, C(H), C(Halogen), C(C₁-C₄-Alkyl), C(C₁-C₄-Halogenalkyl), C(C₁-C₄-Alkoxy) oder C(C₁-C₄-Halogenalkoxy) bedeutet.

8. Verbindung nach einem der Ansprüche 1 bis 7, wobei
R¹ Fluor, Chlor, Brom, Iod, C₁-C₂-Alkyl oder C₁-C₂-Halogenalkyl bedeutet;
R² ein gegebenenfalls substituiertes Phenyl, Pyridyl, Thienyl oder Chinolyl bedeutet;
R³ Fluor, Chlor oder Brom bedeutet;
R⁴ Wasserstoff, Fluor, Chlor, Brom, C₁-C₃-Alkyl, C₁-C₃-Halogenalkyl, C₁-C₃-Alkoxy, C₁-C₃-Halogenalkoxy oder Cyano bedeutet;
R⁵ Fluor, Chlor oder Brom bedeutet; und
X N, C(H), C(Cl), C(F), C(Br), C(l), C(C₁-C₃-Alkyl), C (C₁-C₃-Halogenalkyl), C (C₁-C₃-Alkoxy) oder C (C₁-C₃-Halogenalkoxy) bedeutet.

9. Verbindung nach einem der Ansprüche 1 bis 8, wobei
R¹ Fluor, Chlor, Brom, Methyl oder Ethyl bedeutet;
R² Phenyl, 3-Fluorphenyl, 4-Fluorphenyl, 3-Chlorphenyl, 4-Chlorphenyl, 3-Bromphenyl, 4-Bromphenyl, m-Tolyl, p-Tolyl, 3-Trifluormethylphenyl, 4-Trifluormethylphenyl, 3-Methoxyphenyl, 4-Methoxyphenyl, 3-Trifluormethoxyphenyl, 4-Trifluormethoxyphenyl, 3-Cyanophenyl, 4-Cyanophenyl, 3,4-Difluorphenyl, 3,4-Dichlorphenyl, 3,4-Dimethylphenyl, 3,4-Dimethoxyphenyl, 3-Chlor-4-fluorphenyl, 3-Chlor-4-methylphenyl, 3-Chlor-4-methoxyphenyl, 4-Chlor-3-fluorphenyl, 4-Chlor-3-methylphenyl, 4-Chlor-3-methoxyphenyl, 3-Fluor-4-methoxyphenyl, 3-Fluor-4-methylphenyl, 4-Fluor-3-methoxyphenyl, 4-Fluor-3-methylphenyl, 3-Methoxy-4-methylphenyl, 4-Methoxy-3-methylphenyl, Pyridin-2-yl, Pyridin-3-yl, Pyridin-4-yl, 6-Chlorpyridin-2-yl, 6-Fluorpyridin-2-yl, 6-Methoxypyridin-2-yl, 6-Methylpyridin-2-yl, 6-Chlorpyridin-3-yl, 6-Fluorpyridin-3-yl, 6-Methoxypyridin-3-yl, 6-Methylpyridin-3-yl, 2-Chlorpyridin-4-yl, 2-Fluorpyridin-4-yl, 2-Methoxypyridin-4-yl, 2-Methylpyridin-4-yl, 5-Chlorthiophen-2-yl, 5-Bromthiophen-2-yl, 5-Methoxythiophen-2-yl, Chinolin-2-yl, Chinolin-3-yl, 4-Methoxychinolin-2-yl oder 4-Methylchinolin-2-yl bedeutet;
R³ Fluor oder Chlor bedeutet;
R⁴ Wasserstoff, Fluor, Chlor, C₁-C₂-Alkyl, C₁-C₂-Halogenalkyl, C₁-C₂-Alkoxy, C₁-C₂-Halogenalkoxy oder Cyano bedeutet;
R⁵ Fluor oder Chlor bedeutet; und
X N, C(H), C(Cl), C(F), C(Br), C(C₁-C₂-Alkyl), C (C₁-C₂-Halogenalkyl), C (C₁-C₂-Alkoxy) oder C (C₁-C₂-Halogenalkoxy) bedeutet.

10. Verbindung nach einem der Ansprüche 1 bis 9, wobei
R¹ Fluor, Chlor, Methyl oder Ethyl bedeutet;
R² 4-Fluorphenyl, 4-Chlorphenyl, 4-Bromphenyl, 6-Chlorpyridin-3-yl, 6-Fluorpyridin-3-yl, 6-Methoxypyridin-3-yl, 6-Methylpyridin-3-yl, Chinolin-2-yl, Chinolin-3-yl, 4-Methoxychinolin-2-yl oder 4-Methylchinolin-2-yl bedeutet;
R³ Fluor oder Chlor bedeutet;
R⁴ Wasserstoff, Fluor, Chlor, C₁-C₂-Alkyl, C₁-C₂-Halogenalkyl oder C₁-C₂-Alkoxy bedeutet;
R⁵ Fluor oder Chlor bedeutet; und
X N, C(H), C(C1) oder C(F) bedeutet.

11. Verbindung nach einem der Ansprüche 1 bis 10, wobei
R¹ Chlor oder Methyl bedeutet;
R² 4-Bromphenyl, 6-Chlorpyridin-3-yl oder Chinolin-3-yl bedeutet;
R³ Chlor bedeutet;
R⁴ Chlor bedeutet;
R⁵ Chlor bedeutet; und
X N bedeutet.

12. Verbindung nach Anspruch 1, ausgewählt aus der Reihe
2-Chlor-5-[2,5-dichlor-3-(2,4-6-trifluorphenyl)-3H-imidazol-4-yl]pyridin;
2-Chlor-5-[2-chlor-5-methyl-3-(2,4,6-trifluorphenyl)-3H-imidazol-4-yl]pyridin;
5-[2-Chlor-5-methyl-3-(2,4,6-trifluorphenyl)-3H-imidazol-4-yl]-2-methoxypyridin;
3-[2-Chlor-5-methyl-3-(2,4,6-trifluorphenyl)-3H-imidazol-4-yl]chinolin;
3-[2,5-Dichlor-3-(2,4-6-trifluorphenyl)-3H-imidazol-4-yl]chinolin;
3-[2-Chlor-3-(2,6-difluor-4-methoxyphenyl)-5-methyl-3H-imidazol-4-yl]chinolin;
3-[2,5-Dichlor-3-(2,6-difluor-4-methoxyphenyl)-3H-imidazol-4-yl]chinolin;
2-[2-Chlor-5-methyl-3-(2,4,6-trifluorphenyl)-3H-imidazol-4-yl]chinolin;
2-[2-Chlor-5-methyl-3-(2,4,6-trifluorphenyl)-3H-imidazol-4-yl]-4-methoxychinolin;
2-[2-Chlor-3-(2,6-difluor-4-methoxyphenyl)-5-methyl-3H-imidazol-4-yl]-4-methoxychinolin;
3,5-Dichlor-2-[2,4-dichlor-5-(6-chlorpyridin-3-yl)imidazol-1-yl]pyridin;
3,5-Dichlor-2-[2-chlor-5-(6-chlorpyridin-3-yl)-4-methylimidazol-1-yl]pyridin;
5-[2-Chlor-3-(2,6-difluor-4-methoxyphenyl)-5-methyl-3H-imidazol-4-yl]-2-methylpyridin; und
3,5-Dichlor-2-[2-chlor-5-(4-methoxyphenyl)4-methylimidazol-1-yl]pyridin.

13. Fungizide Zusammensetzung zum Bekämpfen von bzw. den Schutz gegen phytopathogene(n) Mikroorganismen, umfassend als Wirkstoff mindestens eine Verbindung nach einem der Ansprüche 1 bis 12 in freier Form oder in agrarchemisch verwendbarer Salzform, sowie mindestens einen Hilfsstoff.

14. Zusammensetzung nach Anspruch 16, die mindestens eine zusätzliche fungizid wirksame Verbindung, vorzugsweise aus der Gruppe der Azole, Pyrimidinylcarbinole, 2-Aminopyrimidine, Morpholine, Anilinopyrimidine, Pyrrole, Phenylamide, Benzimidazole, Dicarboximide, Carboxamide, Strobilurine, Dithiocarbamate, N-Halogenmethylthiotetrahydrophthalimide, Kupferverbindungen, Nitrophenole, phosphororganische Derivate, Pyridazine, Triazolopyrimidine, Carboxamide oder Benzamide, umfasst.

15. Verwendung einer Verbindung nach einem der Ansprüche 1 bis 12 zum Bekämpfen von bzw. Vorbeugen gegen Befall von Pflanzen, geernteten Nahrungsmittelkulturpflanzen, Samen oder nicht lebenden Materialien durch phytopathogene Mikroorganismen.

16. Verfahren zum Bekämpfen von bzw. Vorbeugen gegen Befall von Kulturpflanzen, geernteten Nahrungsmittelkulturpflanzen oder nicht lebenden Materialien durch phytopathogene Mikroorganismen oder Verderbmikroorganismen oder Organismen, die eine potentielle Gefahr für den Menschen darstellen, bei dem man eine Verbindung nach einem der Ansprüche 1 bis 12 als Wirkstoff auf die Pflanzen, die Pflanzenteile oder ihren Standort, auf Samen oder einen beliebigen Teil der nicht lebenden Materialien aufbringt.

17. Verfahren nach Anspruch 16, wobei es sich bei den phytopathogenen Mikroorganismen um pilzliche Organismen handelt.

## Revendications

1. Composé de formule (I) 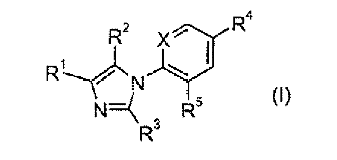 dans laquelle
R¹ est halogène, C₁-C₄alkyle ou C₁-C₄halogénoalkyle ;
R² est un aryle ou hétéroaryle éventuellement substitué ;
R³ est halogène ;
R⁴ est hydrogène, halogène, C₁-C₄alkyle, C₁-C₄halogénoalkyle, C₁-C₄alcoxy, C₁-C₄halogénoalcoxy ou cyano ;
R⁵ est halogène ;
X est N ou C(R) ; et
R est hydrogène, halogène, C₁-C₄alkyle, C₁-C₄halogénoalkyle, C₁-C₄alcoxy, C₁-C₄halogénoalcoxy ou cyano ;
ou une forme sel agrochimiquement utilisable de celui-ci ;
à condition que
lorsque X est C(R), R² ne puisse pas être un aryle éventuellement substitué.

2. Composé selon la revendication 1, **caractérisé en ce que** R¹ est halogène, C₁-C₃alkyle ou C₁-C₃halogénoalkyle.

3. Composé selon l'une ou l'autre des revendications 1 et 2, **caractérisé en ce que** R² est un phényle, naphtyle, pyridyle, thiényle ou quinolyle éventuellement substitué.

4. Composé selon l'une quelconque des revendications 1 à 3, **caractérisé en ce que** R³ est fluoro, chloro, bromo ou iodo.

5. Composé selon l'une quelconque des revendications 1 à 4, **caractérisé en ce que** R⁴ est hydrogène, halogène, C₁-C₃alkyle, C₁-C₃halogénoalkyle, C₁-C₃alcoxy, C₁-C₃halogénoalcoxy ou cyano.

6. Composé selon l'une quelconque des revendications 1 à 5, **caractérisé en ce que** R⁵ est fluoro, chloro, bromo ou iodo.

7. Composé selon l'une quelconque des revendications 1 à 6, **caractérisé en ce que** X est N, C(H), C(halogène), C(C₁-C₄alkyle) , C(C₁-C₄halogénoalkyle), C(C₁-C₄alcoxy) ou C(C₁-C₄halogénoalcoxy).

8. Composé selon l'une quelconque des revendications 1 à 7, **caractérisé en ce que**
R¹ est fluoro, chloro, bromo, iodo, C₁-C₂alkyle ou C₁-C₂halogénoalkyle ;
R² est un phényle, pyridyle, thiényle ou quinolyle éventuellement substitué ;
R³ est fluoro, chloro ou bromo ;
R⁴ est hydrogène, fluoro, chloro, bromo, C₁-C₃alkyle, C₁-C₃halogénoalkyle, C₁-C₃alcoxy, C₁-C₃halogénoalcoxy ou cyano ;
R⁵ est fluoro, chloro ou bromo ; et
X est N, C(H), C(C1), C(F), C(Br), C(1), C(C₁-C₃alkyle), C (C₁-C₃halogénoalkyle), C (C₁-C₃alcoxy) ou C(C₁-C₃halogénoalcoxy).

9. Composé selon l'une quelconque des revendications 1 à 8, **caractérisé en ce que**
R¹ est fluoro, chloro, bromo, méthyle ou éthyle ;
R² est phényle, 3-fluorophényle, 4-fluorophényle, 3-chlorophényle, 4-chlorophényle, 3-bromophényle, 4-bromophényle, m-tolyle, p-tolyle, 3-trifluorométhylphényle, 4-trifluorométhylphényle, 3-méthoxyphényle, 4-méthoxyphényle, 3-trifluorométhoxyphényle, 4-trifluorométhoxyphényle, 3-cyanophényle, 4-cyanophényle, 3,4-difluorophényle, 3,4-dichlorophényle, 3,4-diméthylphényle, 3,4-diméthoxyphényle, 3-chloro-4-fluorophényle, 3-chloro-4-méthylphényle, 3-chloro-4-méthoxyphényle, 4-chloro-3-fluorophényle, 4-chloro-3-méthylphényle, 4-chloro-3-méthoxyphényle, 3-fluoro-4-méthoxyphényle, 3-fluoro-4-méthylphényle, 4-fluoro-3-méthoxyphényle, 4-fluoro-3-méthylphényle, 3-méthoxy-4-méthylphényle, 4-méthoxy-3-méthylphényle, pyridin-2-yle, pyridin-3-yle, pyridin-4-yle, 6-chloropyridin-2-yle, 6-fluoropyridin-2-yle, 6-méthoxypyridin-2-yle, 6-méthylpyridin-2-yle, 6-chloropyridin-3-yle, 6-fluoropyridin-3-yle, 6-méthoxypyridin-3-yle, 6-méthylpyridin-3-yle, 2-chloropyridin-4-yle, 2-fluoropyridin-4-yle, 2-méthoxypyridin-4-yle, 2-méthylpyridin-4-yle, 5-chlorothiophèn-2-yle, 5-bromothiophèn-2-yle, 5-méthoxythiophèn-2-yle, quinoléin-2-yle, quinoléin-3-yle, 4-méthoxyquinoléin-2-yle ou 4-méthylquinoléin-2-yle ;
R³ est fluoro ou chloro ;
R⁴ est hydrogène, fluoro, chloro, C₁-C₂alkyle, C₁-C₂halogénoalkyle, C₁-C₂alcoxy, C₁-C₂halogénoalcoxy ou cyano ;
R⁵ est fluoro ou chloro ; et
X est N, C(H), C(Cl), C(F), C(Br), C(C₁-C₂alkyle), C(C₁-C₂halogénoalkyle), C (C₁-C₂alcoxy) ou C(C₁-C₂halogénoalcoxy).

10. Composé selon l'une quelconque des revendications 1 à 9, **caractérisé en ce que**
R¹ est fluoro, chloro, méthyle ou éthyle ;
R² est 4-fluorophényle, 4-chlorophényle, 4-bromophényle, 6-chloropyridin-3-yle, 6-fluoropyridin-3-yle, 6-méthoxypyridin-3-yle, 6-méthylpyridin-3-yle, quinoléin-2-yle, quinoléin-3-yle, 4-méthoxyquinoléin-2-yle ou 4-méthylquinoléin-2-yle ;
R³ est fluoro ou chloro ;
R⁴ est hydrogène, fluoro, chloro, C₁-C₂alkyle, C₁-C₂halogénoalkyle ou C₁-C₂alcoxy ;
R⁵ est fluoro ou chloro ; et
X est N, C(H), C(Cl) ou C(F).

11. Composé selon l'une quelconque des revendications 1 à 10, **caractérisé en ce que**
R¹ est chloro ou méthyle ;
R² est 4-bromophényle, 6-chloropyridin-3-yle ou quinoléin-3-yle ;
R³ est chloro ;
R⁴ est chloro ;
R⁵ est chloro ; et
X est N.

12. Composé selon la revendication 1, choisi parmi
la 2-chloro-5-[2,5-dichloro-3-(2,4,6-trifluoro-phényl)-3H-imidazol-4-yl]-pyridine ;
la 2-chloro-5-[2-chloro-5-méthyl-3-(2,4,6-trifluoro-phényl)-3H-imidazol-4-yl]-pyridine ;
la 5-[2-chloro-5-méthyl-3-(2,4,6-trifluoro-phényl)-3H-imidazol-4-yl]-2-méthoxy-pyridine ;
la 3-[2-chloro-5-méthyl-3-(2,4,6-trifluoro-phényl)-3H-imidazol-4-yl]-quinoléine ;
la 3-[2,5-dichloro-3-(2,4,6-trifluoro-phényl)-3H-imidazol-4-yl]-quinoléine ;
la 3-[2-chloro-3-(2,6-difluoro-4-méthoxy-phényl)-5-méthyl-3H-imidazol-4-yl]-quinoléine ;
la 3-[2,5-dichloro-3-(2,6-difluoro-4-méthoxy-phényl)-3H -imidazol-4-yl]-quinoléine ;
la 2-[2-chloro-5-méthyl-3-(2,4,6-trifluoro-phényl)-3H-imidazol-4-yl-quinoléine ;
la 2-[2-chloro-5-méthyl-3-(2,4,6-trifluoro-phényl)-3H-imidazol-4-yl]-4-méthoxy-quinoléine ;
la 2-[2-chloro-3-(2,6-difluoro-4-méthoxy-phényl)-5-méthyl-3H-imidazol-4-yl]-4-méthoxy-quinoléine ;
la 3,5-dichloro-2-[2,4-dichloro-5-(6-chloro-pyridin-3-yl)-imidazol-1-yl]-pyridine ;
la 3,5-dichloro-2-[2-chloro-5-(6-chloro-pyridin-3-yl)-4 -méthyl-imidazol-1-yl]-pyridine ;
la 5-[2-chloro-3-(2,6-difluoro-4-méthoxy-phényl)-5-méthyl-3H-imidazol-4-yl]-2-méthyl-pyridine ;
et
la 3,5-dichloro-2-[2-chloro-5-(4-méthoxy-phényl)-4-méthyl-imidazol-1-yl]-pyridine.

13. Composition fongicide destinée à lutter ou à protéger contre les microorganismes phytopathogènes, comprenant, à titre de principe actif, au moins un composé tel que défini dans l'une quelconque des revendications 1 à 12, sous forme libre ou sous forme de sel agrochimiquement utilisable, et au moins un adjuvant.

14. Composition selon la revendication 16, **caractérisée en ce qu'**elle comprend au moins un composé supplémentaire actif du point de vue fongicide, choisi de préférence dans le groupe constitué d'azoles, pyrimidinyl-carbinols, 2-amino-pyrimidines, morpholines, anilinopyrimidines, pyrroles, phénylamides, benzimidazoles, dicarboximides, carboxamides, strobilurines, dithiocarbamates, N-halogénométhylthiotétrahydrophtalimides, composés du cuivre, nitrophénols, dérivés organophosphorés, pyridazines, triazolopyrimidines, carboxamides ou benzamides.

15. Utilisation d'un composé tel que défini dans l'une quelconque des revendications 1 à 12, pour lutter contre ou prévenir l'infestation des plantes, des cultures alimentaires récoltées, des semences ou des matières non vivantes par les microorganismes phytopathogènes.

16. Méthode pour lutter contre ou prévenir une infestation des plantes cultivées, des cultures alimentaires récoltées ou des matières non vivantes par des microorganismes phytopathogènes ou d'altération ou des organismes potentiellement nuisibles pour l'homme, **caractérisée en ce qu'**elle comprend l'application d'un composé tel que défini dans l'une quelconque des revendications 1 à 12, à titre de principe actif, sur les plantes, les parties des plantes ou l'emplacement de celles-ci, sur les semences ou sur une partie quelconque des matières non vivantes.

17. Méthode selon la revendication 16, **caractérisée en ce que** les microorganismes phytopathogènes sont des organismes fongiques.
